(19) **Europäisches Patentamt European Patent Office Office européen des brevets**

(11) **EP 4 703 344 A1**

(12) **EUROPEAN PATENT APPLICATION**
published in accordance with Art. 153(4) EPC

(43) Date of publication:
04.03.2026 Bulletin 2026/10

(21) Application number: 24796758.1

(22) Date of filing: 08.04.2024

(51) International Patent Classification (IPC):
*C07C 51/487* (2006.01)    *C07C 51/43* (2006.01)
*C07C 65/03* (2006.01)

(52) Cooperative Patent Classification (CPC):
(C-Sets available)
C07C 51/02                                      (Cont.)

(86) International application number:
PCT/JP2024/014252

(87) International publication number:
WO 2024/225002 (31.10.2024 Gazette 2024/44)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 25.04.2023 JP 2023071407

(71) Applicant: SUMITOMO BAKELITE CO., LTD.
Tokyo 140-0002 (JP)

(72) Inventors:
• **MURATA, Ryuichi**
**Tokyo 140-0002 (JP)**
• **FUJIWARA, Daisuke**
**Tokyo 140-0002 (JP)**

(74) Representative: **Ter Meer Steinmeister & Partner**
**Patentanwälte mbB**
**Nymphenburger Straße 4**
**80335 München (DE)**

(54) **METHOD FOR PRODUCING 4-HYDROXYBENZOIC ACID**

(57)    An object of the present invention is to provide a method for producing 4-hydroxybenzoic acid, which is capable of producing 4-hydroxybenzoic acid having high purity and less coloring at a high yield. The method for producing 4-hydroxybenzoic acid of the present invention includes: a first acid precipitation step of mixing a starting material liquid containing a metal salt of 4-hydroxybenzoic acid and water with an acid to precipitate a first post-acid-precipitation crystal of 4-hydroxybenzoic acid; a re-dissolution step of mixing the first post-acid-precipitation crystal with a basic solvent to obtain a solution in which the first post-acid-precipitation crystal is re-dissolved, and adding an adsorbent to the solution; a second acid precipitation step of mixing the solution after the re-dissolution step with an acid to precipitate a second post-acid-precipitation crystal of 4-hydroxybenzoic acid; and a first cooling crystallization step of mixing the second post-acid-precipitation crystal with a first aqueous solvent and subsequently performing cooling crystallization to precipitate a first post-crystallization crystal of 4-hydroxybenzoic acid, and separating the first post-crystallization crystal and a first waste liquid.

FIG. 1

EP 4 703 344 A1

(52) Cooperative Patent Classification (CPC): (Cont.)

C-Sets
**C07C 51/02, C07C 65/03;**
**C07C 51/43, C07C 65/03**

**Description**

TECHNICAL FIELD

The present invention relates to a method for producing 4-hydroxybenzoic acid.

BACKGROUND ART

4-Hydroxybenzoic acid is widely used, for example, as a raw material for liquid crystal polymers, phenolic resins, preservatives for cosmetics or pharmaceuticals, and the like. In these applications, since impurities in the starting material affect the applications, it is necessary to purify the starting material sufficiently in advance.

For example, Patent Document 1 discloses a method for producing a cyclic compound, the method including a crystallization step and a solid-liquid separation step. In the crystallization step, a solid having a high purity is precipitated by reducing solubility of a solute in a solution. In the solid-liquid separation step, the precipitated solid is recovered.

Citation List

Patent Document

Patent Document 1: Japanese Unexamined Patent Application No. 2021-038156

SUMMARY OF INVENTION

Technical Problem

In the production method disclosed in Patent Document 1, there is room for improvement from the viewpoint of improving both the purity and the yield. For example, in a case where the purity of the solid to be recovered is to be increased, the efficiency of the crystallization step may be reduced, such as requiring a long time in the crystallization step. On the other hand, in a case where the yield of the solid to be recovered is to be increased, the purity of the solid may be reduced, which may cause unintended coloring.

An object of the present invention is to provide a method for producing 4-hydroxybenzoic acid, which is capable of producing 4-hydroxybenzoic acid having high purity and less coloring at a high yield.

Solution to Problem

Such objects can be achieved by the present invention described in the following items (1) to (10).

(1) A method for producing 4-hydroxybenzoic acid, including:

a first acid precipitation step of mixing a starting material liquid containing a metal salt of 4-hydroxybenzoic acid and water with an acid to precipitate a first post-acid-precipitation crystal of 4-hydroxybenzoic acid;
a re-dissolution step of mixing the first post-acid-precipitation crystal with a basic solvent to obtain a solution in which the first post-acid-precipitation crystal is re-dissolved, and adding an adsorbent to the solution;
a second acid precipitation step of mixing the solution after the re-dissolution step with an acid to precipitate a second post-acid-precipitation crystal of 4-hydroxybenzoic acid; and
a first cooling crystallization step of mixing the second post-acid-precipitation crystal with a first aqueous solvent and subsequently performing cooling crystallization to precipitate a first post-crystallization crystal of 4-hydroxybenzoic acid, and separating the first post-crystallization crystal and a first waste liquid.

(2) The method for producing 4-hydroxybenzoic acid according to (1),
in which, in a case where a process from the first acid precipitation step to the first cooling crystallization step is defined as a first unit process,

the starting material liquid in a first lot is supplied to the first unit process, and subsequently
the starting material liquid in a second lot is supplied to the first unit process,
the first waste liquid obtained from the first unit process to which the starting material liquid in the first lot is supplied is reused as at least one of the basic solvent and the first aqueous solvent used in the first unit process to which the starting material liquid in the second lot is supplied.

(3) The method for producing 4-hydroxybenzoic acid according to (1), further including:
a second cooling crystallization step of mixing the first post-crystallization crystal with a second aqueous solvent and subsequently performing cooling crystallization to precipitate a second post-crystallization crystal of 4-hydroxybenzoic acid, and separating the second post-crystallization crystal and a second waste liquid.

(4) The method for producing 4-hydroxybenzoic acid according to (3),
in which, in a case where a process from the first acid precipitation step to the second cooling crystallization step is defined as a second unit process,

the starting material liquid in a first lot is supplied to the second unit process, and subsequently
the starting material liquid in a second lot is supplied to the second unit process,
the second waste liquid obtained from the second unit process to which the starting material liquid in the first lot is supplied is reused as at least one of the basic solvent and the first aqueous solvent used in the second unit process to which the starting material liquid in the second lot is supplied.

(5) The method for producing 4-hydroxybenzoic acid according to (4),

in which the first waste liquid obtained from the second unit process to which the starting material liquid in the first lot is supplied is reused as the basic solvent used in the second unit process to which the starting material liquid in the second lot is supplied, and
the second waste liquid obtained from the second unit process to which the starting material liquid in the first lot is supplied is reused as the first aqueous solvent used in the second unit process to which the starting material liquid in the second lot is supplied.

(6) The method for producing 4-hydroxybenzoic acid according to any one of (3) to (5), further including:
a third cooling crystallization step of mixing the second post-crystallization crystal with a third aqueous solvent and subsequently performing cooling crystallization to precipitate a third post-crystallization crystal of 4-hydroxybenzoic acid, and separating the third post-crystallization crystal and a third waste liquid.

(7) The method for producing 4-hydroxybenzoic acid according to (3),
in which, in a case where a process from the first acid precipitation step to the third cooling crystallization step is defined as a third unit process,

the starting material liquid in a first lot is supplied to the third unit process, and subsequently
the starting material liquid in a second lot is supplied to the third unit process,
the third waste liquid obtained from the third unit process to which the starting material liquid in the first lot is supplied is reused as at least one of the basic solvent, the first aqueous solvent, and the second aqueous solvent used in the third unit process to which the starting material liquid in the second lot is supplied.

(8) The method for producing 4-hydroxybenzoic acid according to (7),

in which the first waste liquid obtained from the third unit process to which the starting material liquid in the first lot is supplied is reused as the basic solvent used in the third unit process to which the starting material liquid in the second lot is supplied,
the second waste liquid obtained from the third unit process to which the starting material liquid in the first lot is supplied is reused as the first aqueous solvent used in the third unit process to which the starting material liquid in the second lot is supplied, and
the third waste liquid obtained from the third unit process to which the starting material liquid in the first lot is supplied is reused as the second aqueous solvent used in the third unit process to which the starting material liquid in the second lot is supplied.

(9) The method for producing 4-hydroxybenzoic acid according to any one of (1) to (5),
in which, in a case where the first post-crystallization crystal is mixed with ethanol to prepare an ethanol solution having a concentration of 20 g/L, an absorbance of the ethanol solution at a wavelength of 425 nm is 0.025 or less.
(10) The method for producing 4-hydroxybenzoic acid according to any one of (1) to (5),
in which the adsorbent is activated carbon.

Advantageous Effects of Invention

According to the present invention, 4-hydroxybenzoic acid having high purity and less coloring can be produced at a high yield.

BRIEF DESCRIPTION OF DRAWINGS

[FIG. 1] A flowchart for describing a method for producing 4-hydroxybenzoic acid according to a first embodiment.
[FIG. 2] A flowchart for describing a method for producing 4-hydroxybenzoic acid according to a second embodiment.
[FIG. 3] A flowchart for describing a method for producing 4-hydroxybenzoic acid according to a third embodiment.
[FIG. 4] A flowchart for describing a method for producing 4-hydroxybenzoic acid according to a fourth embodiment.
[FIG. 5] A flowchart for describing a method for producing 4-hydroxybenzoic acid according to a fifth embodiment.
[FIG. 6] A flowchart for describing a method for producing 4-hydroxybenzoic acid according to a sixth embodiment.

DESCRIPTION OF EMBODIMENTS

Hereinafter, the method for producing 4-hydroxybenzoic acid according to the present invention will be described in detail based on suitable embodiments shown in the accompanying drawings.

1. First embodiment

First, a method for producing 4-hydroxybenzoic acid according to a first embodiment will be described.

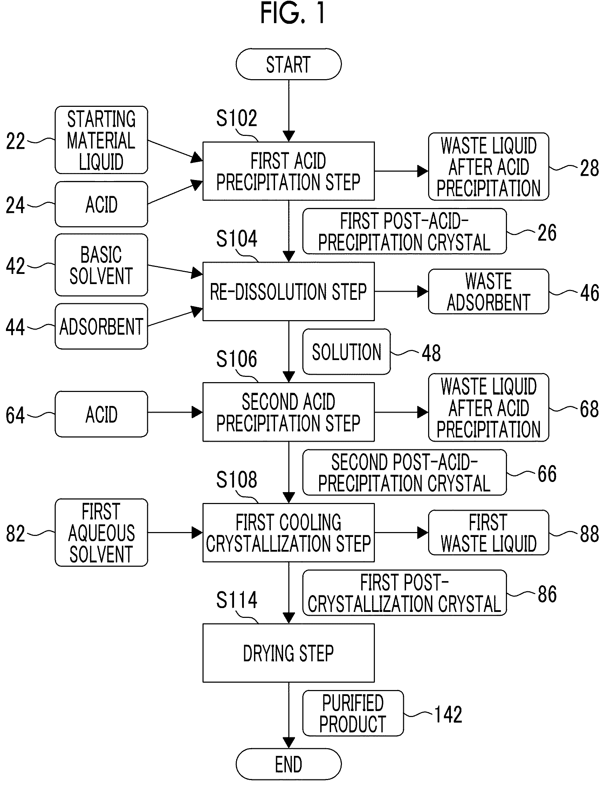

FIG. 1 is a flowchart for describing the method for producing 4-hydroxybenzoic acid according to the first embodiment. In the present specification, the upper side of the flowchart shown in each drawing will be described as "upstream side", and the lower side thereof will be described as "downstream side".
In the method for producing 4-hydroxybenzoic acid according to the present embodiment, 4-hydroxybenzoic acid before purification is purified to recover high-purity 4-hydroxybenzoic acid. The 4-hydroxybenzoic acid is used as a raw material for various applications such as a resin composition, a cosmetic, a pharmaceutical, and a pesticide. In order to realize the application, it is essential to increase the purity of the 4-hydroxybenzoic acid as a raw material.
The method for producing 4-hydroxybenzoic acid shown in FIG. 1 includes a first acid precipitation step S102, a re-dissolution step S104, a second acid precipitation step S106, a first cooling crystallization step S108, and a drying step S114. Hereinafter, each step will be sequentially described.

1.1. First acid precipitation step

In the first acid precipitation step S102, first, a starting material liquid 22 is prepared in a container. The starting material liquid 22 contains the 4-hydroxybenzoic acid before purification. Hereinafter, the 4-hydroxybenzoic acid before purification is referred to as "unpurified 4HBA", and 4-hydroxybenzoic acid after purification is referred to as "purified 4HBA". The 4HBA refers to the 4-hydroxybenzoic acid.
The unpurified 4HBA may be a compound derived from a fossil resource, or may be a compound derived from biomass. The biomass refers to plant-derived organic resources. Specific examples thereof include a resource stored in a form of a sugar such as starch, glucose, and cellulose, a body of an animal which grows by eating a plant body, and a product obtained by processing a plant body or an animal body. By using a compound derived from biomass as the unpurified 4HBA, since the purified 4HBA is not derived from a fossil resource, it is possible to contribute to suppression of an increase in carbon dioxide.
For example, a fermentation production process using a microorganism is preferably used for crude purification of the unpurified 4HBA. That is, it is preferable that the starting material liquid 22 includes a fermentation solution. In addition, it is preferable that the starting raw material to be subjected to the fermentation is a sugar. Since the sugar, particularly the monosaccharide is present in large amounts in nature, it is easily available.
A concentration of the unpurified 4HBA in the starting material liquid 22 is not particularly limited, but is preferably 0.5% by mass or more and 15.0% by mass or less, more preferably 1.0% by mass or more and 14.0% by mass or less, and still more preferably 2.0% by mass or more and 13.0% by mass or less. Even in a case where such a starting material liquid 22 having a relatively low concentration is used, the present embodiment can purify the 4HBA with an excellent yield.
In addition, a content of water in the starting material liquid 22 is not particularly limited, but is preferably 50% by mass or more, and more preferably 75% by mass or more. In the present embodiment, even in a case where the starting material liquid 22 contains water in such a proportion, the starting material liquid 22 can be used without being concentrated. Therefore, the present embodiment is useful from the viewpoint of reducing man-hours required for the purification of the 4HBA. In addition, in a case where the starting material liquid 22 is the fermentation solution, the starting material liquid 22 generally contains water at the above-described content. In the present embodiment, since such the fermentation solution can also be applied as it is to the starting material liquid 22, usability is favorable. In a case where the starting material liquid 22 includes the fermentation solution, various organic acids other than the 4HBA, metal salts thereof, or the like may be contained in the starting material liquid 22 as by-products of the fermentation.

The 4HBA contained in the starting material liquid 22 may be contained in a form of various salts such as an alkali metal salt. In a case where the starting material liquid 22 includes the fermentation solution, the 4HBA is usually present in a form of the salt. Examples of an alkali metal contained in the alkali metal salt include sodium and potassium. Hereinafter, a case where the 4HBA is present in a form of the alkali metal salt will be described as an example.

Next, in the first acid precipitation step S102, an acid 24 is added to the starting material liquid 22 in the container. As a result, the alkali metal salt of the 4HBA contained in the starting material liquid 22 reacts with the acid 24 to generate an acid salt of the alkali metal. In addition, the starting material liquid 22 is in a state in which the 4HBA coexists with the acid salt of the alkali metal. In a case where a concentration of the acid 24 with respect to the starting material liquid 22 increases, a pH of the starting material liquid 22 decreases and solubility of the 4HBA decreases, so that an abundance ratio of the 4HBA increases. As a result, crystals of the 4HBA are precipitated in the starting material liquid 22. The crystals of the 4HBA precipitated in the first acid precipitation step S102 are referred to as "first post-acid-precipitation crystals 26". On the other hand, the acid salt of the alkali metal dissolves in the starting material liquid 22 due to the decrease in the pH. The acid salt of the alkali metal is an impurity, and is removed by solid-liquid separation described later.

Examples of the acid 24 include an inorganic acid and an organic acid. Examples of the inorganic acid include hydrochloric acid, nitric acid, sulfuric acid, phosphoric acid, and perchloric acid. Examples of the organic acid include formic acid and acetic acid. The acid 24 may be a mixture of two or more of these acids. Among the above, as the acid 24, from the viewpoint of ease of handling, cost, and the like, hydrochloric acid or sulfuric acid is preferably used, and sulfuric acid is more preferably used.

In a case where the acid 24 is, for example, sulfuric acid, an alkali metal sulfate is generated in the starting material liquid 22. The alkali metal sulfate is present in a dissolved state in the starting material liquid 22. Hereinafter, a case where the acid salt of the alkali metal is the alkali metal sulfate will be described as an example.

The pH of the starting material liquid 22 after the addition of the acid 24 is preferably 1.5 or more and 4.0 or less, more preferably 2.0 or more and 3.5 or less, and still more preferably 2.5 or more and 3.0 or less. As a result, the first post-acid-precipitation crystals 26 can be sufficiently precipitated in the starting material liquid 22, and the solubility of the alkali metal sulfate can be increased. In a case where the pH is lower than the above-described lower limit value, the solubility of the alkali metal sulfate in the starting material liquid 22 may be reduced, or chemical resistance of the container may be adversely affected. On the other hand, in a case where the pH is higher than the above-described upper limit value, a precipitation efficiency of the first post-acid-precipitation crystals 26 may be reduced.

A temperature of the starting material liquid 22 after the addition of the acid 24 is not particularly limited, but is preferably 5°C or higher and 40°C or lower, and more preferably 10°C or higher and 30°C or lower. As a result, the precipitation efficiency of the first post-acid-precipitation crystals 26 can be increased. In addition, the solubility of the alkali metal sulfate can be appropriately secured.

The starting material liquid 22 to which the acid 24 has been added may be stirred as necessary. Any stirring device may be used for the stirring.

After the addition of the acid 24, the precipitated first post-acid-precipitation crystals 26 are recovered by solid-liquid separation. Examples of a method of the solid-liquid separation include centrifugation, centrifugal filtration, filter press filtration, and membrane filtration. As a result, the first post-acid-precipitation crystals 26 as a solid phase and a waste liquid 28 after the acid precipitation as a liquid phase are separated from each other.

1.2. Re-dissolution step

In the re-dissolution step S104, the first post-acid-precipitation crystals 26, a basic solvent 42, and an adsorbent 44 are put into a container to prepare a mixture. As a result, the first post-acid-precipitation crystals 26 are dissolved in the basic solvent 42. In addition, an adsorption treatment is performed on the mixture by the adsorbent 44.

Examples of the basic solvent 42 include an aqueous solution of a base. Examples of the base include sodium hydroxide, potassium hydroxide, gaseous ammonia, potassium carbonate, sodium carbonate, and sodium bicarbonate; and one or two or more of these may be used in combination. The base is added to water to prepare the basic solvent 42. Examples of the water include pure water, distilled water, and ion exchange water; and pure water is particularly preferably used.

An addition amount and a concentration of the basic solvent 42 are set such that the first post-acid-precipitation crystals 26 are completely dissolved in the mixture. A pH of the mixture prepared by the addition of the basic solvent 42 is preferably 5.5 or more, more preferably 6.0 or more and 9.0 or less, and still more preferably 6.5 or more and 7.5 or less. As a result, the first post-acid-precipitation crystals 26 can be sufficiently dissolved, and the loss due to the adsorption of the 4HBA can be suppressed.

A temperature of the mixture in which the first post-acid-precipitation crystals 26 are dissolved is not particularly limited, but is preferably 20°C or higher and 80°C or lower, and more preferably 25°C or higher and 70°C or lower. As a result, the solubility of the first post-acid-precipitation crystals 26 in the mixture can be sufficiently increased while suppressing the thermal decomposition of the 4HBA, and thus the work efficiency can be improved.

In addition, the adsorbent 44 adsorbs the hydrophobic impurities in the mixture without adsorbing the dissolved 4HBA. In addition, the hydrophobic impurities may include a coloring-causative component. The adsorbent 44 can remove the coloring-causative component in the mixture while suppressing the loss of the 4HBA. As a result, the purified 4HBA is finally obtained as crystals with less coloring and high purity.

As the adsorbent 44, any adsorbent can be used, and examples thereof include activated carbon, hydrophobic zeolite, hydrophobic silica gel, hydrophobic alumina gel, hydrophobic silica-alumina gel, activated clay, molecular sieve, and carbon molecular sieve. Since these adsorbents have hydrophobicity, the hydrophobic impurities can be efficiently adsorbed and removed. In addition, activated carbon is preferably used as the adsorbent 44. Since the activated carbon has a characteristic that is not easily changed even when in contact with water for a long time and has favorable dispersibility in water, the activated carbon is useful as the adsorbent 44.

The mixture containing the adsorbent 44 may be stirred as necessary. Any stirring device may be used for the stirring.

An amount of the adsorbent 44 used in the re-dissolution step S104 is not particularly limited, but is preferably 0.001 kg or more, more preferably 0.01 kg or more and 0.50 kg or less, still more preferably 0.02 kg or more and 0.40 kg or less, and particularly preferably 0.03 kg or more and 0.40 kg or less with respect to 1 kg of the first post-acid-precipitation crystals 26 in terms of dry weight. As a result, the impurities can be efficiently adsorbed to the adsorbent 44, and the purity of the 4HBA can be sufficiently increased.

In a case where the amount of the adsorbent 44 is lower than the above-described lower limit value, the adsorbent 44 may be insufficient, and thus the function thereof may not be sufficiently exhibited. On the other hand, in a case where the amount of the adsorbent 44 is higher than the above-described upper limit value, there is a concern that an excess adsorbent 44 is generated, which causes an increase in cost, or that adsorption failure due to the adsorbent 44 occurs due to occurrence of stirring unevenness.

The adsorbent 44 after the treatment is removed as a waste adsorbent 46 by solid-liquid separation such as filtration. In a case where the waste adsorbent 46 is removed, a solution 48 in which the 4HBA is dissolved is obtained.

In addition to the above-described adsorbents, an ion exchange resin may be used in combination with the adsorbent 44. By using the ion exchange resin, the adsorbent 44 can efficiently adsorb ionic impurities. Therefore, the purity of the 4HBA can be further increased.

Examples of the ionic impurities which can be removed by the adsorbent 44 include $F^-$, $Cl^-$, $NO_2^-$, $Br^-$, $NO_3^-$, $PO_4^{3-}$, $SO_4^{2-}$, $(COO)_2^{2-}$, $CH_3COO^-$, $HCOO^-$, $Li^+$, $Na^+$, $NH_4^+$, $K^+$, $Mg^{2+}$, and $Ca^{2+}$.

By performing the re-dissolution step S104 after the first acid precipitation step S102 as described above, decolorization operation can be performed on the first post-acid-precipitation crystals 26 after the impurities such as the alkali metal sulfate are removed. As a result, the coloring-causative component can be efficiently removed, and finally, the crystals of the 4HBA with less coloring can be produced.

1.3. Second acid precipitation step

In the second acid precipitation step S106, an acid 64 is added to the solution 48 in the container. As a result, crystals of the 4HBA are precipitated in the solution 48. The crystals of the 4HBA precipitated in the second acid precipitation step S106 are referred to as "second post-acid-precipitation crystals 66". In addition, the acid salt of the alkali metal, which is the impurity, is sufficiently dissolved in the solution 48.

Examples of the acid 64 include various inorganic acids and organic acids exemplified as the acid 24. Among the above, as the acid 64, from the viewpoint of ease of handling, cost, and the like, hydrochloric acid or sulfuric acid is preferably used, and sulfuric acid is more preferably used.

A pH of the solution 48 after the addition of the acid 64 is preferably 1.5 or more and 4.0 or less, more preferably 2.0 or more and 3.5 or less, and still more preferably 2.5 or more and 3.0 or less. As a result, the second post-acid-precipitation crystals 66 can be sufficiently precipitated in the solution 48, and the solubility of the impurities can be increased.

A temperature of the solution 48 after the addition of the acid 64 is not particularly limited, but is preferably 5°C or higher and 40°C or lower, and more preferably 10°C or higher and 30°C or lower. As a result, the precipitation efficiency of the second post-acid-precipitation crystals 66 can be increased. In addition, the solubility of the impurities can be appropriately secured.

The solution 48 to which the acid 64 has been added may be stirred as necessary. Any stirring device may be used for the stirring.

After the addition of the acid 64, the precipitated second post-acid-precipitation crystals 66 are recovered by solid-liquid separation. Examples of a method of the solid-liquid separation include centrifugation, centrifugal filtration, filter press filtration, and membrane filtration. As a result, the second post-acid-precipitation crystals 66 as a solid phase and a waste liquid 68 after the acid precipitation as a liquid phase are separated from each other.

By providing such a second acid precipitation step S106, impurities which cannot be removed in the first acid precipitation step S102, for example, the acid salt of the alkali metal, such as the alkali metal sulfate, can be further removed. In addition, in the second acid precipitation step S106, the second post-acid-precipitation crystals 66 as a solid having a sufficiently suppressed content of impurities can be supplied to a first cooling crystallization step S108 described later, which utilizes a state change of a solid and a liquid. Therefore, the second acid precipitation step S106 contributes to efficiently recovering high-purity crystals in the end.

1.4. First cooling crystallization step

In the first cooling crystallization step S108, the second post-acid-precipitation crystals 66 are subjected to a cooling crystallization treatment. The cooling crystallization treatment is a treatment including a first operation and a second operation. The first operation is an operation of adding a first aqueous solvent 82 to the second post-acid-precipitation crystals 66 and heating the obtained mixture to dissolve the second post-acid-precipitation crystals 66. The second operation is an operation of cooling the mixture to precipitate the 4HBA.

A heating temperature of the mixture in the operation (first operation) of dissolving the second post-acid-precipitation crystals 66 is appropriately adjusted depending on a pressure in the container, but is preferably 70°C or higher and 200°C or lower and more preferably 80°C or higher and 150°C or lower. As a result, the second post-acid-precipitation crystals 66 can be sufficiently dissolved in the first aqueous solvent 82. As a result, the yield of the final 4HBA crystals can be increased.

In a case where the heating temperature is lower than the above-described lower limit value, it may take time to dissolve the second post-acid-precipitation crystals 66 or a part thereof may not be dissolved and remain. On the other hand, in a case where the heating temperature is higher than the above-described upper limit value, the impurities may also crystallize in the present step, and thus the purity of the final 4HBA crystals may be reduced.

In the cooling crystallization treatment, the pressure may be kept constant, or the pressure may be changed. For example, the operation (first operation) of dissolving the second post-acid-precipitation crystals 66 may be performed under pressure. As a result, the solubility of the 4HBA can be increased even in a case where the heating temperature is lowered. The pressure during pressurization is preferably 0.2 MPa or more and 2.0 MPa or less, and more preferably 0.3 MPa or more and 1.5 MPa or less.

The first aqueous solvent 82 is a solvent containing water as a main component. Examples of the water include pure water, distilled water, and ion exchange water; and pure water is particularly preferably used. The first aqueous solvent 82 may contain various water-soluble organic solvents, in addition to the water. Examples of the water-soluble organic solvent include lower alcohols such as methanol, ethanol, and isopropanol, and ketones such as acetone, methyl ethyl ketone, methyl propyl ketone, methyl isobutyl ketone, diisobutyl ketone, and cyclohexanone.

A content of the water in the first aqueous solvent 82 is preferably 70% by mass or more, more preferably 80% by mass or more, and still more preferably 100% by mass. As a result, the cost of the first aqueous solvent 82 can be suppressed.

An amount of the first aqueous solvent 82 used is not particularly limited, but is preferably 2 parts by mass or more and 20 parts by mass or less, and more preferably 4 parts by mass or more and 10 parts by mass or less with respect to 1 part by mass of the second post-acid-precipitation crystals 66. As a result, the second post-acid-precipitation crystals 66 can be sufficiently dissolved in the first aqueous solvent 82, and an amount of a first waste liquid 88 generated in the present step can be suppressed.

In the second operation, the mixture is cooled. The cooling may be natural cooling or forced cooling. By the cooling, the solubility of the 4HBA in the mixture is reduced, and the crystals of 4HBA can be precipitated. A reaching temperature after the cooling is not particularly set, but is set to approximately room temperature, for example, 10°C or higher and 30°C or lower. The crystals of the 4HBA precipitated in the operation are referred to as "first post-crystallization crystals 86". In the cooling process, impurities are discharged from the crystal structure with the crystal growth of the 4HBA. Therefore, in the first post-crystallization crystals 86, the amount of impurities is particularly suppressed to be small.

A cooling rate of the mixture is preferably 0.05 °C/min or more and 3.0 °C/min or less, and more preferably 0.1 °C/min or more and 2.0 °C/min or less. As a result, the impurities are less likely to be incorporated into the precipitated crystals, and the crystals of the 4HBA having a higher purity can be precipitated.

In the process of the operation of cooling the mixture, the mixture may be stirred as necessary. Any stirring device may be used for the stirring.

The precipitated first post-crystallization crystals 86 are recovered by solid-liquid separation. Examples of a method of the solid-liquid separation include centrifugation, centrifugal filtration, filter press filtration, and membrane filtration. As a result, the first post-crystallization crystals 86 as a solid phase and the first waste liquid 88 as a liquid phase are separated from each other. The first waste liquid 88 contains impurities which are not completely removed in each of the steps on the upstream side of the present step, for example, the ionic impurities.

By performing the first cooling crystallization step S108 as described above, the crystallization of the 4HBA can be performed in the presence of the first aqueous solvent 82. Since the first aqueous solvent 82 contains water as a main component, it contains almost no component which can be an impurity. Therefore, in the present step, high-purity crystals of the 4HBA can be obtained. In addition, in the cooling crystallization, supersaturation of the 4HBA in the mixture is adjusted mainly by adjusting the temperature. Therefore, the adjustment of the supersaturation is easy, and as a result, the control of the crystal particle diameter can be easily performed. As a result, the crystals of 4HBA having a desired crystal particle diameter can be obtained. The control of the crystal particle diameter is useful in that handleability of the product finally obtained is improved.

In addition, the second post-acid-precipitation crystals 66 obtained through the first acid precipitation step S102, the re-dissolution step S104, and the second acid precipitation step S106 are supplied to the present step. Since the second post-acid-precipitation crystals 66 are crystals obtained by removing various impurities in each of the steps, controllability of the crystal particle diameter in the present step is easily improved in association with the adjustment of the supersaturation. Therefore, in the present step, crystals having a large crystal particle diameter and a small amount of fine crystals, that is, crystals having high handleability can be easily obtained.

1.5. Drying step

In the drying step S114, the first post-crystallization crystals 86 are subjected to a drying treatment. The drying treatment is a treatment for removing the liquid contained in the first post-crystallization crystals 86. As a result of the treatment, for example, crystals of 4HBA in a form of powder, granules, or the like are obtained. The crystals are recovered as a purified product 142 shown in FIG. 1.

Examples of the drying treatment include a treatment accompanied by an operation such as a heating operation, a decompression operation, and a gas blowing operation.

Here, a temperature during the heating operation is not particularly limited, but is preferably 40°C to 90°C, more preferably 50°C to 85°C, and still more preferably 60°C to 80°C.

In addition, a pressure during the heating operation is not particularly limited and may be atmospheric pressure, but is preferably below atmospheric pressure (101 kPa), more preferably 90 kPa or less, and still more preferably 80 kPa or less. As a result, drying at a relatively low temperature is possible, and thus it is easy to suppress thermal denaturation of the 4HBA. The lower limit value of the pressure may not be set, but is preferably 20 kPa or more in a case of considering decompression cost and the like.

A heating time is not particularly limited, but is preferably 1 to 120 minutes and more preferably 5 to 90 minutes.

In the drying step S114, all of the liquids contained in the first post-crystallization crystals 86 may not be removed, and the purified product 142 may be left with the liquid as necessary.

In addition, the drying step S114 may be provided as necessary, and may not be performed in a case where the purified product 142 in a state of leaving the liquid as described above is to be recovered.

2. Second embodiment

Next, a method for producing 4-hydroxybenzoic acid according to a second embodiment will be described.

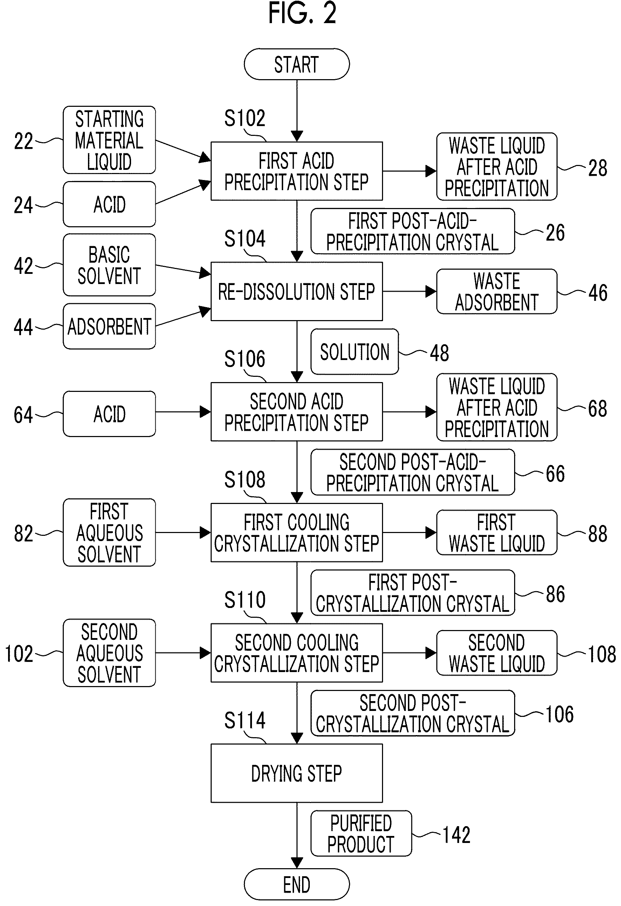

FIG. 2 is a flowchart for describing the method for producing 4-hydroxybenzoic acid according to the second embodiment.

Hereinafter, the second embodiment will be described, but in the following description, the differences from the first embodiment will be mainly described, and the same matters will not be repeated.

The second embodiment is the same as the first embodiment, except that a second cooling crystallization step S110 is added.

As shown in FIG. 2, the second cooling crystallization step S110 is provided between the first cooling crystallization step S108 and the drying step S114. In the second cooling crystallization step S110, the first post-crystallization crystals 86 are subjected to a cooling crystallization treatment. The cooling crystallization treatment is the same as the treatment performed in the first cooling crystallization step S108 described above. That is, the cooling crystallization treatment in the second cooling crystallization step S110 is a treatment of performing the same operation as the first and second operations. Specifically, the same operation as the first operation is an operation of adding a second aqueous solvent 102 to the first post-crystallization crystals 86 and heating the obtained mixture to dissolve second post-crystallization crystals 106. The same operation as the second operation is an operation of cooling the mixture to precipitate the 4HBA and recovering second post-crystallization crystals 106.

The second post-crystallization crystals 106 are recovered by solid-liquid separation. In the solid-liquid separation, the second post-crystallization crystals 106 as a solid phase and a second waste liquid 108 as a liquid phase are separated from each other.

The second aqueous solvent 102 is a solvent containing water as a main component. Examples of the water include pure water, distilled water, and ion exchange water; and pure water is particularly preferably used. The second aqueous solvent 102 may contain various water-soluble organic solvents, in addition to the water. Examples of the water-soluble organic solvent include lower alcohols such as methanol, ethanol, and isopropanol, and ketones such as acetone, methyl ethyl ketone, methyl propyl ketone, methyl isobutyl ketone, diisobutyl ketone, and cyclohexanone.

A content of the water in the second aqueous solvent 102 is preferably 70% by mass or more, more preferably 80% by mass or more, and still more preferably 100% by mass. As a result, the cost of the second aqueous solvent 102 can be suppressed.

An amount of the second aqueous solvent 102 used is not particularly limited, but is preferably 2 parts by mass or more and 20 parts by mass or less, and more preferably 4 parts by mass or more and 10 parts by mass or less with respect to 1 part by mass of the first post-crystallization crystals 86. As a result, the first post-crystallization crystals 86 can be sufficiently dissolved in the second aqueous solvent 102, and an amount of the second waste liquid 108 generated in the present step can be suppressed.

By performing the second cooling crystallization step S110 as described above, the crystallization of the 4HBA can be performed in the presence of the second aqueous solvent 102. Since the second aqueous solvent 102 contains water as a main component, it contains almost no component which can be an impurity. Therefore, in the present step, crystals of 4HBA having a higher purity can be obtained. In addition, in the cooling crystallization, supersaturation of the 4HBA in the mixture is adjusted mainly by adjusting the temperature. Therefore, the adjustment of the supersaturation is easy, and as a result, the control of the crystal particle diameter can be easily performed. As a result, the crystals of 4HBA having a desired crystal particle diameter can be obtained. The control of the crystal particle diameter is useful in that handleability of the product finally obtained is improved.

In addition, since the first post-crystallization crystals 86 to be used in the present step are crystals obtained by removing various impurities in each of the steps, controllability of the crystal particle diameter in the present step is further easily improved in association with the adjustment of the supersaturation. Therefore, in the present step, crystals having a large crystal particle diameter and a small amount of fine crystals, that is, crystals having high handleability can be more easily obtained.

In the second embodiment as described above, the same effects as those in the first embodiment can be obtained.

3. Third embodiment

Next, a method for producing 4-hydroxybenzoic acid according to a third embodiment will be described.

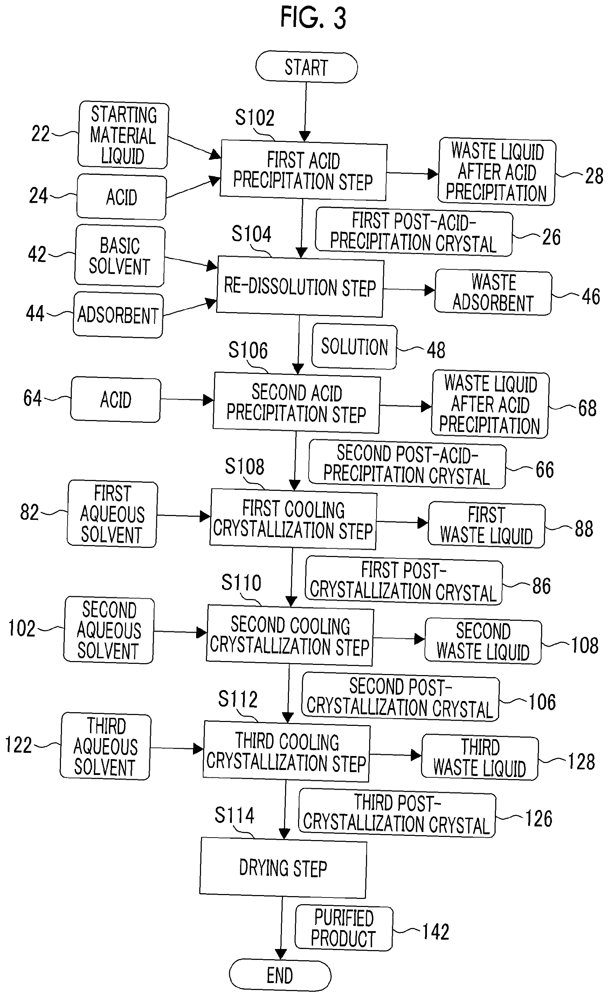

FIG. 3 is a flowchart for describing the method for producing 4-hydroxybenzoic acid according to the third embodiment.

Hereinafter, the third embodiment will be described, but in the following description, the differences from the second embodiment will be mainly described, and the same matters will not be repeated.

The third embodiment is the same as the second embodiment, except that a third cooling crystallization step S112 is added.

As shown in FIG. 3, the third cooling crystallization step S112 is provided between the second cooling crystallization step S110 and the drying step S114. In the third cooling crystallization step S112, the second post-crystallization crystals 106 are subjected to a cooling crystallization treatment. The cooling crystallization treatment is the same as the treatment performed in the first cooling crystallization step S108 described above. That is, the cooling crystallization treatment in the third cooling crystallization step S112 is a treatment of performing the same operation as the first and second operations. Specifically, the same operation as the first operation is an operation of adding a third aqueous solvent 122 to the second post-crystallization crystals 106 and heating the obtained mixture to dissolve the second post-crystallization crystals 106. The same operation as the second operation is an operation of cooling the mixture to precipitate the 4HBA and recovering third post-crystallization crystals 126.

The third post-crystallization crystals 126 are recovered by solid-liquid separation. In the solid-liquid separation, the third post-crystallization crystals 126 as a solid phase and a third waste liquid 128 as a liquid phase are separated from each other.

The third aqueous solvent 122 is a solvent containing water as a main component. Examples of the water include pure water, distilled water, and ion exchange water; and pure water is particularly preferably used. The third aqueous solvent 122 may contain various water-soluble organic solvents, in addition to the water. Examples of the water-soluble organic solvent include lower alcohols such as methanol, ethanol, and isopropanol, and ketones such as acetone, methyl ethyl ketone, methyl propyl ketone, methyl isobutyl ketone, diisobutyl ketone, and cyclohexanone.

A content of the water in the third aqueous solvent 122 is preferably 70% by mass or more, more preferably 80% by mass or more, and still more preferably 100% by mass. As a result, the cost of the third aqueous solvent 122 can be suppressed.

An amount of the third aqueous solvent 122 used is not particularly limited, but is preferably 2 parts by mass or more and 20 parts by mass or less, and more preferably 4 parts by mass or more and 10 parts by mass or less with respect to 1 part by mass of the second post-crystallization crystals 106. As a result, the second post-crystallization crystals 106 can be sufficiently dissolved in the third aqueous solvent 122, and an amount of the third waste liquid 128 generated in the present step can be suppressed.

By performing the third cooling crystallization step S112 as described above, the crystallization of the 4HBA can be performed in the presence of the third aqueous solvent 122. Since the third aqueous solvent 122 contains water as a main component, it contains almost no component which can be an impurity. Therefore, in the present step, crystals of 4HBA having a higher purity can be obtained. In addition, in the cooling crystallization, supersaturation of the 4HBA in the mixture is adjusted mainly by adjusting the temperature. Therefore, the adjustment of the supersaturation is easy, and as a result, the control of the crystal particle diameter can be easily performed. As a result, the crystals of 4HBA having a desired crystal particle diameter can be obtained. The control of the crystal particle diameter is useful in that handleability of the product finally obtained is improved.

In addition, since the second post-crystallization crystals 106 to be used in the present step are crystals obtained by removing various impurities in each of the steps, controllability of the crystal particle diameter in the present step is further easily improved in association with the adjustment of the supersaturation. Therefore, in the present step, crystals having a large crystal particle diameter and a small amount of fine crystals, that is, crystals having high handleability can be more easily obtained.

In the third embodiment as described above, the same effects as those in the second embodiment can be obtained. In addition, the step of performing the same cooling crystallization treatment as in the third cooling crystallization step S112 may be further performed one or more times. As a result, the purity of the 4HBA can be further increased.

4. Fourth embodiment

Next, a method for producing 4-hydroxybenzoic acid according to a fourth embodiment will be described.

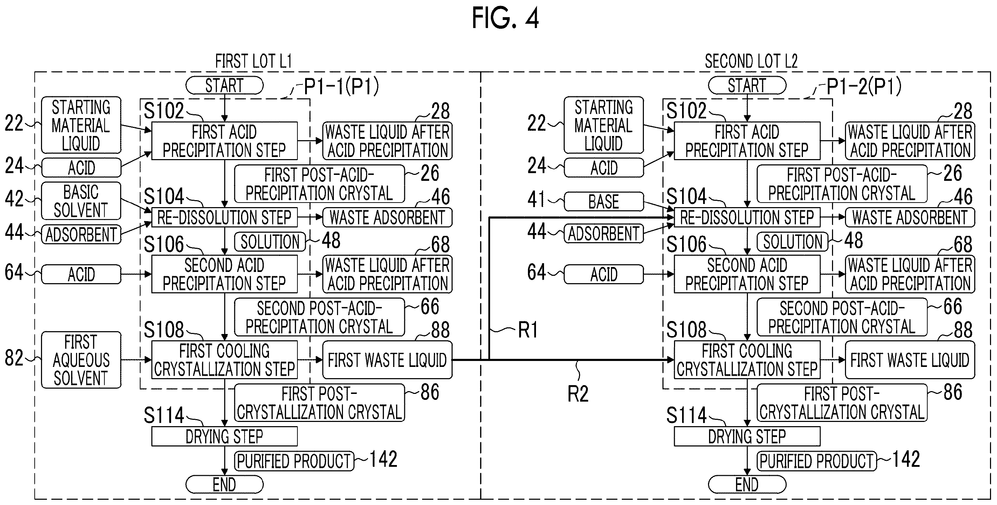

FIG. 4 is a flowchart for describing the method for producing 4-hydroxybenzoic acid according to the fourth embodiment.

Hereinafter, the fourth embodiment will be described, but in the following description, the differences from the first embodiment will be mainly described, and the same matters will not be repeated.

The fourth embodiment is the same as the first embodiment, except that, in a case where each of starting material liquids 22 in different lots are sequentially subjected to the production method according to the first embodiment to obtain a purified product 142 of each lot, the first waste liquid 88 generated in the process of purifying the starting material liquid 22 in the preceding lot is reused in the process of purifying the starting material liquid 22 of the next lot.

FIG. 4 shows a method of obtaining the purified product 142 by supplying the starting material liquid 22 in a first lot L1 to a first unit process P1 and the drying step S114, and then obtaining the purified product 142 additionally by supplying the starting material liquid 22 in a second lot L2 to the first unit process P1 and the drying step S114. In the following description, the first unit process P1 using the starting material liquid 22 in the first lot L1 will be also referred to as "first unit process P1-1"; and the first unit process P1 using the starting material liquid 22 in the second lot L2 will be also referred to as "first unit process P1-2". In addition, the first unit process P1 refers to the first acid precipitation step S102 to the first cooling crystallization step S108 included in the first embodiment.

In the first unit process P1-1, the first waste liquid 88 is generated in the first cooling crystallization step S108. In the present embodiment, the first waste liquid 88 is reused in the first unit process P1-2. Specifically, the first waste liquid 88 is reused in at least one of the basic solvent 42 and the first aqueous solvent 82, used in the first unit process P1-2. The reuse in the present embodiment refers to performing any one or both of a reuse operation R1 of replacing a part or all of the basic solvent 42 with the first waste liquid 88 and a reuse operation R2 of replacing a part or all of the first aqueous solvent 82 with the first waste liquid 88. By performing at least one of the reuse operation R1 and the reuse operation R2, the 4HBA contained in the first waste liquid 88 can be used in the first unit process P1-2. As a result, the yield of the 4HBA in the first unit process P1-2 can be increased. In addition, it is possible to reduce the environmental load due to the discharge and disposal of the first waste liquid 88.

In the reuse operation R1, it is preferable to replace 30% by mass or more of the basic solvent 42 with the first waste liquid 88; it is more preferable to replace 50% by mass or more of the basic solvent 42 with the first waste liquid 88; and it is still more preferable to replace 70% by mass or more of the basic solvent 42 with the first waste liquid 88. As a result, the yield of the 4HBA can be particularly increased, and the amount of the new basic solvent 42, which is to be used unless the reuse operation R1 is performed, can be sufficiently reduced. Similarly, in the reuse operation R2, it is preferable to replace 30% by mass or more of the first aqueous solvent 82 with the first waste liquid 88; it is more preferable to replace 50% by mass or more of the first aqueous solvent 82 with the first waste liquid 88; and it is still more preferable to replace 70% by mass or more of the first aqueous solvent 82 with the first waste liquid 88. As a result, the yield of the 4HBA can be particularly increased, and the amount of the new first aqueous solvent 82, which is to be used unless the reuse operation R2 is performed, can be sufficiently reduced.

In addition, by the reuse as described above, the amount of the basic solvent 42 and the amount of the first aqueous solvent 82 used in the first unit process P1-2 can be reduced. As a result, it is possible to reduce the running cost.

In FIG. 4, a movement path of the first waste liquid 88 by the reuse operations R1 and R2 (the step in which the first waste liquid 88 is reused) is indicated by an arrow. In a case where the reuse operation R1 is performed, a base 41 shown in FIG. 4 may be added to the first waste liquid 88. As a result, the first waste liquid 88 can be adjusted to be basic.

In addition, the first waste liquid 88 generated in the first unit process P1-2 may be reused in the first unit process using the starting material liquid 22 in third or subsequent lots (not shown).

The fourth embodiment also exhibits the same effect as that of the first embodiment, in addition to the effects as described above.

5. Fifth embodiment

Next, a method for producing 4-hydroxybenzoic acid according to a fifth embodiment will be described.

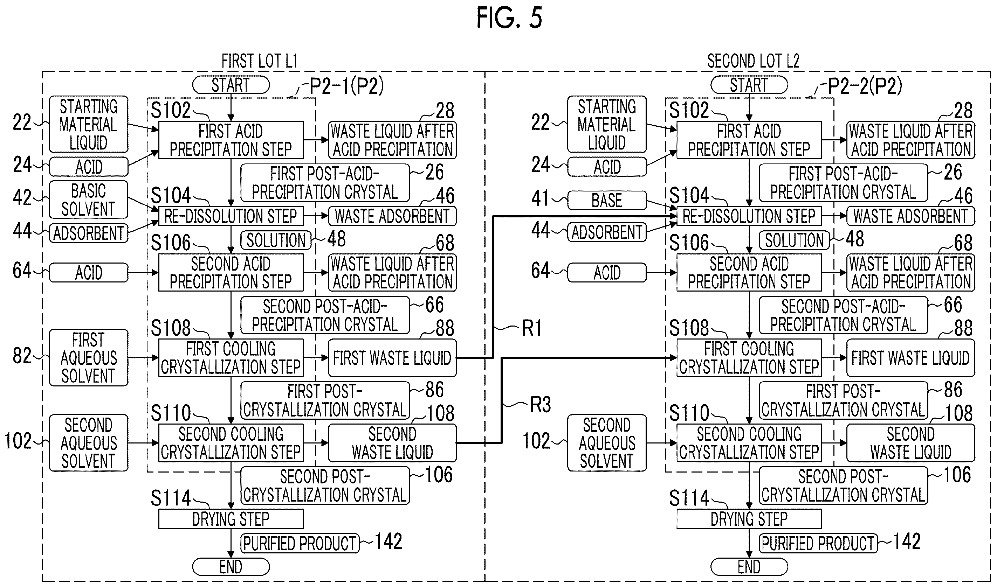

FIG. 5 is a flowchart for describing the method for producing 4-hydroxybenzoic acid according to the fifth embodiment.

Hereinafter, the fifth embodiment will be described, but in the following description, the differences from the second embodiment will be mainly described, and the same matters will not be repeated.

The fifth embodiment is the same as the second embodiment, except that, in a case where each of starting material liquids 22 in different lots are sequentially subjected to the production method according to the second embodiment to obtain a purified product 142 of each lot, the first waste liquid 88 and the second waste liquid 108, generated in the process of purifying the starting material liquid 22 in the preceding lot, are reused in different processes.

FIG. 5 shows a method of obtaining the purified product 142 by supplying the starting material liquid 22 in a first lot L1 to a second unit process P2 and the drying step S114, and then obtaining the purified product 142 additionally by supplying the starting material liquid 22 in a second lot L2 to the second unit process P2 and the drying step S114. In the following description, the second unit process P2 using the starting material liquid 22 in the first lot L1 will be also referred to as "second unit process P2-1"; and the second unit process P2 using the starting material liquid 22 in the second lot L2 will be also referred to as "second unit process P2-2". In addition, the second unit process P2 refers to the first acid precipitation step S102 to the second cooling crystallization step S110 included in the second embodiment.

In the second unit process P2-1, the first waste liquid 88 is generated in the first cooling crystallization step S108. In the present embodiment, the first waste liquid 88 is reused in the re-dissolution step S104 of the second unit process P2-2. In addition, the second waste liquid 108 is generated in the second cooling crystallization step S110 in the second unit process P2-1. In the present embodiment, the second waste liquid 108 is reused in the first cooling crystallization step S108 of the second unit process P2-2. The reuse in the present embodiment refers to performing both of a reuse operation R1 of replacing a part or all of the basic solvent 42 used in the re-dissolution step S104 with the first waste liquid 88 and a reuse operation R3 of replacing a part or all of the first aqueous solvent 82 used in the first cooling crystallization step S108 with the second waste liquid 108. By performing both of the reuse operations R1 and R3, the 4HBA contained in the first waste liquid 88 and the second waste liquid 108 can be used in the second unit process P2-2. As a result, the yield of the 4HBA in the second unit process P2-2 can be increased. In addition, it is possible to reduce the environmental load due to the discharge and disposal of the first waste liquid 88 or the second waste liquid 108.

In the reuse operation R1, it is preferable to replace 30% by mass or more of the basic solvent 42 with the first waste liquid 88; it is more preferable to replace 50% by mass or more of the basic solvent 42 with the first waste liquid 88; and it is still more preferable to replace 70% by mass or more of the basic solvent 42 with the first waste liquid 88. As a result, the yield of the 4HBA can be particularly increased, and the amount of the new basic solvent 42, which is to be used unless the reuse operation R1 is performed, can be sufficiently reduced. Similarly, in the reuse operation R3, it is preferable to replace 30% by mass or more of the first aqueous solvent 82 with the second waste liquid 108; it is more preferable to replace 50% by mass or more of the first aqueous solvent 82 with the second waste liquid 108; and it is still more preferable to replace 70% by mass or more of the first aqueous solvent 82 with the second waste liquid 108. As a result, the yield of the 4HBA can be particularly increased, and the amount of the new first aqueous solvent 82, which is to be used unless the reuse operation R3 is performed, can be sufficiently reduced.

In addition, since the second waste liquid 108 is generated from a step downstream of the first waste liquid 88, the concentration of impurities is lower and the relative contamination degree is lower than that of the first waste liquid 88. Therefore, by reusing the first waste liquid 88 in the re-dissolution step S104 and reusing the second waste liquid 108 in the first cooling crystallization step S108, which is a step downstream of the re-dissolution step S104, the amount of impurities carried into the crystals of the 4HBA due to the reuse can be minimized. Specifically, a contamination degree of the first waste liquid 88 is higher than that of the second waste liquid 108. However, by using the first waste liquid 88 in the re-dissolution step S104 located relatively on the upstream side, the probability that the impurities are carried into the final purified product 142 can be kept low. On the other hand, a contamination degree of the second waste liquid 108 is lower than that of the first waste liquid 88. Therefore, even in a case where the second waste liquid 108 is used in the first cooling crystallization step S108 downstream of the re-dissolution step S104, the probability that the impurities are carried into the purified product 142 is low. Therefore, in the present embodiment, the yield of the 4HBA can be increased by reusing both the first waste liquid 88 and the second waste liquid 108, while being capable of suppressing the decrease in purity.

In addition, by the reuse as described above, the amount of the basic solvent 42 and the amount of the first aqueous solvent 82 used in the second unit process P2-2 can be reduced. As a result, it is possible to reduce the running cost.

In FIG. 5, a movement path of the first waste liquid 88 by the reuse operation R1 and a movement path of the second waste liquid 108 by the reuse operation R3 are indicated by arrows. In a case where the reuse operation R1 is performed, a base 41 shown in FIG. 5 may be added to the first waste liquid 88. As a result, the first waste liquid 88 can be adjusted to be basic.

In addition, although not shown in FIG. 5, a reuse operation of replacing a part or all of the basic solvent 42 with the second waste liquid 108 may be performed. That is, the second waste liquid 108 may be reused as at least one of the basic solvent 42 and the first aqueous solvent 82. Even in this case, the effect of increasing the yield of the 4HBA in the second unit process P2-2 is obtained.

In addition, at least one of the first waste liquid 88 and the second waste liquid 108, generated in the second unit process P2-2, may be reused in the second unit process using the starting material liquid 22 in third or subsequent lots (not shown).

The fifth embodiment also exhibits the same effect as that of the second embodiment, in addition to the effects as described above.

6. Sixth embodiment

Next, a method for producing 4-hydroxybenzoic acid according to a sixth embodiment will be described.

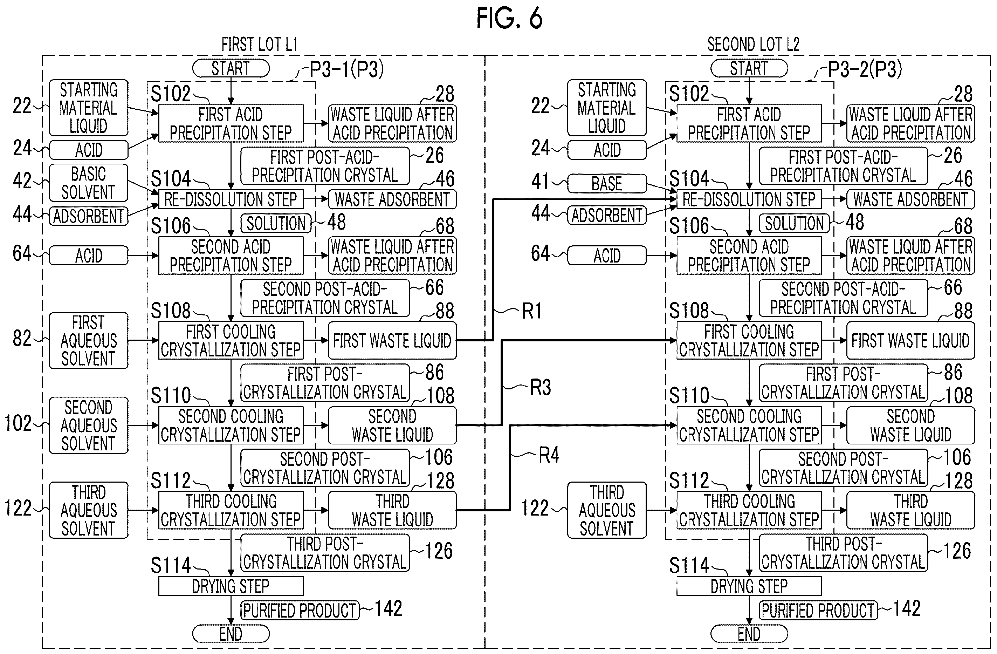

FIG. 6 is a flowchart for describing the method for producing 4-hydroxybenzoic acid according to the sixth embodiment.

Hereinafter, the sixth embodiment will be described, but in the following description, the differences from the third embodiment will be mainly described, and the same matters will not be repeated.

The sixth embodiment is the same as the third embodiment, except that, in a case where each of starting material liquids 22 in different lots are sequentially subjected to the production method according to the third embodiment to obtain a purified product 142 of each lot, the first waste liquid 88, the second waste liquid 108, and the third waste liquid 128, generated in the process of purifying the starting material liquid 22 in the preceding lot, are reused in different processes.

FIG. 6 shows a method of obtaining the purified product 142 by supplying the starting material liquid 22 in a first lot L1 to a third unit process P3 and the drying step S114, and then obtaining the purified product 142 additionally by supplying the starting material liquid 22 in a second lot L2 to the third unit process P3 and the drying step S114. In the following description, the third unit process P3 using the starting material liquid 22 in the first lot L1 will be also referred to as "third unit process P3-1"; and the third unit process P3 using the starting material liquid 22 in the second lot L2 will be also referred to as "third unit process P3-2". In addition, the third unit process P3 refers to the first acid precipitation step S102 to the third cooling crystallization step S112 included in the third embodiment.

In the third unit process P3-1, the first waste liquid 88 is generated in the first cooling crystallization step S108. In the present embodiment, the first waste liquid 88 is reused in the re-dissolution step S104 of the third unit process P3-2. In addition, the second waste liquid 108 is generated in the second cooling crystallization step S110 in the third unit process P3-1. In the present embodiment, the second waste liquid 108 is reused in the first cooling crystallization step S108 of the third unit process P3-2. Furthermore, the third waste liquid 128 is generated in the third cooling crystallization step S112 in the third unit process P3-1. In the present embodiment, the third waste liquid 128 is reused in the second cooling crystallization step S110 of the third unit process P3-2. The reuse in the present embodiment refers to performing all of a reuse operation R1 of replacing a part or all of the basic solvent 42 used in the re-dissolution step S104 with the first waste liquid 88, a reuse operation R3 of replacing a part or all of the first aqueous solvent 82 used in the first cooling crystallization step S108 with the second waste liquid 108, and a reuse operation R4 of replacing a part or all of the second aqueous solvent 102 used in the second cooling crystallization step S110 with the third waste liquid 128. By performing all of the reuse operations R1, R3, and R4, the 4HBA contained in the first waste liquid 88, the second waste liquid 108, and the third waste liquid 128 can be used in the third unit process P3-2. As a result, the yield of the 4HBA in the third unit process P3-2 can be increased. In addition, it is possible to reduce the environmental load due to the discharge and disposal of the first waste liquid 88, the second waste liquid 108, and the third waste liquid 128.

In the reuse operation R1, it is preferable to replace 30% by mass or more of the basic solvent 42 with the first waste liquid 88; it is more preferable to replace 50% by mass or more of the basic solvent 42 with the first waste liquid 88; and it is still more preferable to replace 70% by mass or more of the basic solvent 42 with the first waste liquid 88. As a result, the yield of the 4HBA can be particularly increased, and the amount of the new basic solvent 42, which is to be used unless the reuse operation R1 is performed, can be sufficiently reduced. Similarly, in the reuse operation R3, it is preferable to replace 30% by mass or more of the first aqueous solvent 82 with the second waste liquid 108; it is more preferable to replace 50% by mass or more of the first aqueous solvent 82 with the second waste liquid 108; and it is still more preferable to replace 70% by mass or more of the first aqueous solvent 82 with the second waste liquid 108. As a result, the yield of the 4HBA can be particularly increased, and the amount of the new first aqueous solvent 82, which is to be used unless the reuse operation R3 is performed, can be sufficiently reduced. Similarly, in the reuse operation R4, it is preferable to replace 30% by mass or more of the second aqueous solvent 102 with the third waste liquid 128; it is more preferable to replace 50% by mass or more of the second aqueous solvent 102 with the third waste liquid 128; and it is still more preferable to replace 70% by mass or more of the second aqueous solvent 102 with the third waste liquid 128. As a result, the yield of the 4HBA can be particularly increased, and the amount of the new second aqueous solvent 102, which is to be used unless the reuse operation R4 is performed, can be sufficiently reduced.

In addition, since the third waste liquid 128 is generated from a step downstream of the second waste liquid 108, and the second waste liquid 108 is generated from a step downstream of the first waste liquid 88, concentrations of impurities in both of the third waste liquid 128 and the second waste liquid 108 are relatively low, and the relative contamination degrees are low. Therefore, by reusing the first waste liquid 88 in the re-dissolution step S104, reusing the second waste liquid 108 in the first cooling crystallization step S108, which is a step downstream of the re-dissolution step S104, and reusing the third waste liquid 128 in the second cooling crystallization step S110, which is a step downstream of the re-dissolution step S104, the amount of impurities carried into the crystals of the 4HBA due to the reuse can be minimized. That is, although the first waste liquid 88 has a lower contamination degree than the second waste liquid 108, by using the first waste liquid 88 in the re-dissolution step S104 located relatively on the upstream side, the probability that the impurities are carried into the final purified product 142 can be kept low. On the other hand, since the second waste liquid 108 has a lower contamination degree than the first waste liquid 88, and the third waste liquid 128 has a lower contamination degree than the second waste liquid 108, the probability that the impurities are carried into the purified product 142 is low even in the first cooling crystallization step S108 or the second cooling crystallization step S110, which is downstream of the re-dissolution step S104. Therefore, in the present embodiment, the yield of the 4HBA can be increased by reusing all of the first waste liquid 88, the second waste liquid 108, and the third waste liquid 128, while being capable of suppressing the decrease in purity.

In addition, by the reuse as described above, the amount of the basic solvent 42, the amount of the first aqueous solvent 82, and the amount of the second aqueous solvent 102 used in the third unit process P3-2 can be reduced. As a result, it is possible to reduce the running cost.

In FIG. 6, a movement path of the first waste liquid 88 by the reuse operation R1, a movement path of the second waste liquid 108 by the reuse operation R3, and a movement path of the third waste liquid 128 by the reuse operation R4 are indicated by arrows. In a case where the reuse operation R1 is performed, a base 41 shown in FIG. 6 may be added to the first waste liquid 88. As a result, the first waste liquid 88 can be adjusted to be basic.

In addition, although not shown in FIG. 6, a reuse operation of replacing a part or all of the basic solvent 42 with the third waste liquid 128 may be performed, or a reuse operation of replacing a part or all of the first aqueous solvent 82 with the third waste liquid 128 may be performed. That is, the third waste liquid 128 may be reused as at least one of the basic solvent 42, the first aqueous solvent 82, and the second aqueous solvent 102. Even in this case, the effect of increasing the yield of the 4HBA in the third unit process P3-2 is obtained.

In addition, at least one of the first waste liquid 88, the second waste liquid 108, and the third waste liquid 128, generated in the third unit process P3-2, may be reused in the third unit process using the starting material liquid 22 in third or subsequent lots (not shown).

The sixth embodiment also exhibits the same effect as that of the third embodiment, in addition to the effects as described above.

7. Purified product

7.1. Purity of 4HBA in purified product

In the crystals of the purified 4HBA (purified product 142) in a dried state, a concentration of alkali metal ions is preferably 3,000 ppm or less, more preferably 2,000 ppm or less, and still more preferably 1,000 ppm or less. In the purified product 142 in which the concentration of the alkali metal ions is within the above-described range, the concentration of the ionic impurities is sufficiently low, and thus the purity is sufficiently high. Examples of the alkali metal ions include a sodium ion and a potassium ion.

In addition, in the purified product 142 in a dried state, a concentration of anions derived from the acids 24 and 64 is preferably 6,000 ppm or less, more preferably 5,000 ppm or less, and still more preferably 4,000 ppm or less. In the purified product 142 in which the concentration of the anions derived from the acids 24 and 64 is within the above-described range, the introduction of the anions derived from the acids 24 and 64 is suppressed, and the concentration of the ionic impurities is sufficiently low, and thus the purity is sufficiently high. Examples of the anions derived from the acids 24 and 64 include a chloride ion and a sulfate ion.

A purity of the 4HBA in the purified product 142 in a dried state is preferably 95% or more, more preferably 97% or more, and still more preferably 98% or more. The 4HBA having such a purity is useful as various raw materials because the purity is sufficiently high. The purity of the 4HBA is a ratio of the mass of the 4HBA in the purified product 142 to the total mass of the purified product 142.

The above-described concentration of the ionic impurities is measured by high performance liquid chromatography (HPLC) under the following conditions.

Column: (Cation) Shim-pack IC-C4 (manufactured by Shimadzu Corporation); (Anion) Shim-pack IC-SA2 (manufactured by Shimadzu Corporation)
Mobile phase:

$$\text{Mobile phase: (Cation) water/oxalic acid dihydrate} = 1000/0.315 \text{ (mass/mass)};$$

(Anion) water/sodium hydrogen carbonate/sodium carbonate = 1000/1.008/0.0636 (mass/mass/mass), both anion and cation eluted isocratically

Flow rate: (Both cation and anion) 1 mL/mmin
Column temperature: (Cation) 40°C; (Anion) 30°C
Detection method: electrical conductivity detector

In addition, the concentration of the 4HBA (mass of the 4HBA) is measured by high performance liquid chromatography under the following conditions.

Apparatus: Chromaster (registered trademark) manufactured by Hitachi High-Tech Corporation
Column: COSMOSIL 5C18-AR-II ($\varphi$ 4.6 mm $\times$ 250 mm), manufactured by NACALAI TESQUE, INC.

The "dry state" means a state in which a moisture content in the purified product 142 is 1.0% by mass or less. The moisture content is measured by a Karl Fischer method defined in JIS K 0068:2001.

7.2. Evaluation of coloring of purified product

In addition, in a case where the purified product 142 is added to ethanol to prepare an ethanol solution having a concentration of 20 g/L, an absorbance of the obtained ethanol solution at a wavelength of 425 nm is preferably 0.025 or less, and more preferably 0.020 or less. The purified product 142 in which the absorbance of the ethanol solution is within the above-described range is less colored. Therefore, the purified product 142 is useful as various raw materials.

The absorbance of the ethanol solution is measured as follows using a spectrophotometer.

First, the above-described ethanol solution is put into a quartz cell having an optical path length of 10 mm. Next, a transmittance $T_{425}$ (%) at a wavelength of 425 nm is measured using a spectrophotometer. An absorbance $A_{425}$ is obtained by the following expression (A).

$$A_{425} = -\log(T_{425}/100) \ ... \ (A)$$

The purified product 142 may be a purified product obtained from the first post-crystallization crystals 86, may be a purified product obtained from the second post-crystallization crystals 106, or may be a purified product obtained from the third post-crystallization crystals 126.

7.3. Powder properties of purified product

An aerated bulk density of the purified product 142 is preferably 0.40 g/cm$^3$ or more and 0.85 g/cm$^3$ or less, more preferably 0.45 g/cm$^3$ or more and 0.80 g/cm$^3$ or less, and still more preferably 0.50 g/cm$^3$ or more and 0.75 g/cm$^3$ or less. In a case where the aerated bulk density of the purified product 142 is within the above-described range, a purified product 142 having favorable filling properties and flowability is obtained. Such a purified product 142 is useful as various raw materials because of excellent handleability. In a case where the aerated bulk density is lower than the above-described lower limit value, the filling properties and flowability of the purified product 142 are decreased, and thus the handleability may be decreased. On the other hand, the aerated bulk density may be higher than the above-described upper limit value; but in this case, in the production, additional work such as adding a classification treatment is required, which may increase the cost. Therefore, the production difficulty may be increased.

The aerated bulk density is a bulk density measured in a state in which the purified product 142 which has passed through a sieve having an opening of 1.0 mm is gently filled in a predetermined container. In addition, as a device for measuring the aerated bulk density, a powder tester PT-X manufactured by Hosokawa Micron Group can be used.

In addition, an average crystallite diameter of the purified product 142 is preferably 10 nm or more and 150 nm or less, more preferably 30 nm or more and 120 nm or less, and still more preferably 40 nm or more and 100 nm or less. In a case where the average crystallite diameter of the purified product 142 is within the above-described range, a purified product 142 having high purity and a relatively uniform particle diameter is obtained. Such a purified product 142 is useful as various raw materials because of high purity and excellent handleability. In a case where the average crystallite diameter is lower than the above-described lower limit value, the particle diameter of the powder of the purified product 142 is also decreased, and thus there is a concern that a problem in handleability such as easy scattering may occur. On the other hand, the average crystallite diameter may be higher than the above-described upper limit value; but in this case, the production may take a long time.

The average crystallite diameter of the purified product 142 is calculated from an X-ray diffraction pattern and Scherrer's equation.

8. Effects of embodiments described above

As described above, the method for producing 4-hydroxybenzoic acid according to the above-described embodiment includes the first acid precipitation step S102, the re-dissolution step S104, the second acid precipitation step S106, the first cooling crystallization step S108. In the first acid precipitation step S102, the starting material liquid 22 containing a metal salt of 4-hydroxybenzoic acid and water is mixed with the acid 24 to precipitate the first post-acid-precipitation crystals 26 of 4HBA. In the re-dissolution step S104, the first post-acid-precipitation crystals 26 are mixed with the basic solvent 42 for re-dissolving the first post-acid-precipitation crystals 26 to obtain the solution 48, and then the adsorbent 44 is added to the solution 48. In the second acid precipitation step S106, the solution 48 after the re-dissolution step S104 is mixed with the acid 64 to precipitate second post-acid-precipitation crystals 66 of 4HBA. In the first cooling crystallization step S108, the second post-acid-precipitation crystals 66 are mixed with the first aqueous solvent 82, and then the cooling crystallization is performed to precipitate first post-crystallization crystals 86 of 4HBA and to separate the first post-crystallization crystals 86 and the first waste liquid 88 from each other.

With such a configuration, the impurities are adsorbed between the two acid precipitations, and then the cooling crystallization treatment is performed, whereby 4-hydroxybenzoic acid having high purity and less coloring can be produced at a high yield.

In addition, a process from the first acid precipitation step S102 to the first cooling crystallization step S108 may be defined as the first unit process P1; and the starting material liquid 22 in the first lot L1 may be supplied to the first unit process P1-1, and then the starting material liquid 22 in the second lot L2 may be supplied to the first unit process P1-2. In this case, the first waste liquid 88 obtained from the first unit process P1-1 to which the starting material liquid 22 in the first lot L1 is supplied may be reused as at least one of the basic solvent 42 and the first aqueous solvent 82 used in the first unit process P1-2 to which the starting material liquid 22 in the second lot L2 is supplied.

With such a configuration, the 4HBA contained in the first waste liquid 88 can be used in the first unit process P1-2 to which the starting material liquid 22 in the second lot L2 is supplied. As a result, the yield of the 4HBA can be increased. In addition, it is possible to reduce the environmental load due to the discharge and disposal of the first waste liquid 88.

In addition, the method for producing 4-hydroxybenzoic acid according to the above-described embodiment may include the second cooling crystallization step S110. In the second cooling crystallization step S110, the first post-crystallization crystals 86 are mixed with the second aqueous solvent 102, and then the cooling crystallization is performed to precipitate second post-crystallization crystals 106 of 4HBA and to separate the second post-crystallization crystals 106 and the second waste liquid 108 from each other.

With such a configuration, by performing the cooling crystallization treatment twice, crystals of 4HBA having a higher purity can be obtained.

In addition, a process from the first acid precipitation step S102 to the second cooling crystallization step S110 may be defined as the second unit process P2; and the starting material liquid 22 in the first lot L1 may be supplied to the second unit process P2-1, and then the starting material liquid 22 in the second lot L2 may be supplied to the second unit process P2-2. In this case, the second waste liquid 108 obtained from the second unit process P2-1 to which the starting material liquid 22 in the first lot L1 is supplied may be reused as at least one of the basic solvent 42 and the first aqueous solvent 82 used in the second unit process P2-2 to which the starting material liquid 22 in the second lot L2 is supplied.

With such a configuration, the 4HBA contained in the second waste liquid 108 can be used in the second unit process P2-2 to which the starting material liquid 22 in the second lot L2 is supplied. As a result, the yield of the 4HBA can be increased. In addition, it is possible to reduce the environmental load due to the discharge and disposal of the second waste liquid 108.

In addition, the first waste liquid 88 obtained from the second unit process P2-1 to which the starting material liquid 22 in the first lot L1 is supplied may be reused as the basic solvent 42 used in the second unit process P2-2 to which the starting material liquid 22 in the second lot L2 is supplied. Furthermore, the second waste liquid 108 obtained from the second unit process P2-1 to which the starting material liquid 22 in the first lot L1 is supplied may be reused as the first aqueous solvent 82 used in the second unit process P2-2 to which the starting material liquid 22 in the second lot L2 is supplied.

With such a configuration, the amount of impurities carried into the crystals of 4HBA due to the reuse can be minimized. As a result, the yield of the 4HBA can be increased by reusing both the first waste liquid 88 and the second waste liquid 108, while being capable of suppressing the decrease in purity.

In addition, the method for producing 4-hydroxybenzoic acid according to the above-described embodiment may include the third cooling crystallization step S112. In the third cooling crystallization step S112, the second post-crystallization crystals 106 are mixed with the third aqueous solvent 122, and then the cooling crystallization is performed to precipitate third post-crystallization crystals 126 of 4HBA and to separate the third post-crystallization crystals 126 and the third waste liquid 128 from each other.

With such a configuration, by performing the cooling crystallization treatment three times, crystals of 4HBA having a higher purity can be obtained.

In addition, a process from the first acid precipitation step S102 to the third cooling crystallization step S112 may be defined as the third unit process P3; and the starting material liquid 22 in the first lot L1 may be supplied to the third unit process P3-1, and then the starting material liquid 22 in the second lot L2 may be supplied to the third unit process P3-2. In this case, the third waste liquid 128 obtained from the third unit process P3-1 to which the starting material liquid 22 in the first lot L1 is supplied may be reused as at least one of the basic solvent 42, the first aqueous solvent 82, and the second aqueous solvent 102, used in the third unit process P3-2 to which the starting material liquid 22 in the second lot L2 is supplied.

With such a configuration, the 4HBA contained in the third waste liquid 128 can be used in the third unit process P3-2 to which the starting material liquid 22 in the second lot L2 is supplied. As a result, the yield of the 4HBA can be increased. In addition, it is possible to reduce the environmental load due to the discharge and disposal of the third waste liquid 128.

In addition, the first waste liquid 88 obtained from the third unit process P3-1 to which the starting material liquid 22 in the first lot L1 is supplied may be reused as the basic solvent 42 used in the third unit process P3-2 to which the starting material liquid 22 in the second lot L2 is supplied. Furthermore, the second waste liquid 108 obtained from the third unit process P3-1 to which the starting material liquid 22 in the first lot L1 is supplied may be reused as the first aqueous solvent 82 used in the third unit process P3-2 to which the starting material liquid 22 in the second lot L2 is supplied. Moreover, the third waste liquid 128 obtained from the third unit process P3-1 to which the starting material liquid 22 in the first lot L1 is supplied may be reused as the second aqueous solvent 102 used in the third unit process P3-2 to which the starting material liquid 22 in the second lot L2 is supplied.

With such a configuration, the amount of impurities carried into the crystals of 4HBA due to the reuse can be minimized. As a result, the yield of the 4HBA can be increased by reusing all of the first waste liquid 88, the second waste liquid 108, and the third waste liquid 128, while being capable of suppressing the decrease in purity.

In addition, in a case where the first post-crystallization crystals 86 are mixed with ethanol to prepare an ethanol solution having a concentration of 20 g/L, an absorbance of the ethanol solution at a wavelength of 425 nm is preferably 0.025 or less.

With such a configuration, it is possible to produce crystals of 4HBA with less coloring.

In addition, the adsorbent 44 is preferably activated carbon.

Since the activated carbon has a characteristic that is not easily changed even when in contact with water for a long time and has favorable dispersibility in water, the activated carbon is useful as the adsorbent 44.

Although the method for producing 4-hydroxybenzoic acid according to the embodiments of the present invention has been described above, the present invention is not limited to the embodiments. For example, the method for producing 4-hydroxybenzoic acid according to the embodiment of the present invention may be a method for producing 4-hydroxybenzoic acid to which any step is added to the embodiment.

[Examples]

Next, specific examples of the present invention will be described.

9. Production of purified product

### 9.1. Example 1

A purified product consisting of purified 4HBA was obtained by sequentially performing the steps shown in FIG. 1. The specific details will be described below.

First, a fermentation solution containing unpurified 4HBA was prepared as a starting material liquid. A concentration of sodium 4-hydroxybenzoate in the starting material liquid was 8.0% by mass, and a content of water was 86% by mass. Next, sulfuric acid was added to the starting material liquid. A pH of the starting material liquid after the addition of sulfuric acid was 2.8, and a temperature of the starting material liquid was 25°C. Thereafter, first post-acid-precipitation crystals were recovered by solid-liquid separation (first acid precipitation step).

Next, a basic solvent was added to the recovered first post-acid-precipitation crystals such that a content was 25% by mass on a dry basis, and then powdered activated carbon (manufactured by FUJIFILM Wako Pure Chemical Corporation, activated carbon, powder) was added thereto as an adsorbent. As a base of the basic solvent, sodium hydroxide was used, and as a solvent, ultrapure water was used. A pH of the mixture prepared by the addition of the basic solvent was 7.0, and a temperature of the mixture was 25°C. "% by mass" (or mass) on a dry basis refers to % by mass (or mass) in a case of drying a liquid substance. In addition, an amount of the adsorbent used was 0.04 kg with respect to 1 kg of the first post-acid-precipitation crystals (on a dry basis). Thereafter, a solution was recovered by solid-liquid separation (re-dissolution step).

Next, sulfuric acid was added to the recovered solution. A pH of the solution after the addition of sulfuric acid was 2.8, and a temperature of the solution was 25°C. Thereafter, second post-acid-precipitation crystals were recovered by solid-liquid separation (second acid precipitation step).

Next, pure water (first aqueous solvent) was added to the recovered second post-acid-precipitation crystals to prepare a mixture at 13% by mass on a dry basis, and the obtained mixture was heated to 90°C. Next, the heated mixture was cooled to 25°C. As a result, first post-crystallization crystals were precipitated in the mixture. A cooling rate of the mixture was 0.4 °C/min. Thereafter, first post-crystallization crystals and a first waste liquid were recovered by solid-liquid separation (first cooling crystallization step).

Next, the recovered first post-crystallization crystals were heated and dried to obtain a powdery purified product (drying step).

### 9.2. Example 2

Among the steps shown in FIG. 4, each step using the starting materials in the second lot was sequentially performed to obtain a purified product consisting of purified 4HBA. The specific details will be described below.

First, the same step as the first acid precipitation step of Example 1 was performed to recover first post-acid-precipitation crystals.

Next, the same step as the re-dissolution step of Example 1 was performed to recover a solution. The first waste liquid recovered in the first cooling crystallization step of Example 1 was reused as the basic solvent. Specifically, by performing the reuse operation R1 shown in FIG. 4, the entire amount of the basic solvent, which was originally planned to be used in Example 2, excluding the base, was replaced with the first waste liquid.

Next, the same step as the second acid precipitation step of Example 1 was performed to recover second post-acid-precipitation crystals.

Next, the same step as the first cooling crystallization step of Example 1 was performed to recover first post-crystallization crystals.

Next, the same step as the drying step of Example 1 was performed to obtain a powdery purified product.

### 9.3. Example 3

Among the steps shown in FIG. 4, each step using the starting materials in the second lot was sequentially performed to obtain a purified product consisting of purified 4HBA. The specific details will be described below.

First, the same step as the first acid precipitation step of Example 1 was performed to recover first post-acid-precipitation crystals.

Next, the same step as the re-dissolution step of Example 1 was performed to recover a solution. The first waste liquid recovered in the first cooling crystallization step of Example 1 was reused as the basic solvent. Specifically, by performing the reuse operation R1 shown in FIG. 4, the entire amount of the basic solvent, which was originally planned to be used in Example 3, excluding the base, was replaced with the first waste liquid.

Next, the same step as the second acid precipitation step of Example 1 was performed to recover second post-acid-precipitation crystals.

Next, the same step as the first cooling crystallization step of Example 1 was performed to recover first post-crystallization crystals. The first waste liquid recovered in the first cooling crystallization step of Example 1 was reused as the first aqueous solvent. Specifically, by performing the reuse operation R2 shown in FIG. 4, a part of the first aqueous solvent, which was originally planned to be used in Example 3, was replaced with the first waste liquid.

Next, the same step as the drying step of Example 1 was performed to obtain a powdery purified product.

9.4. Example 4

A purified product consisting of purified 4HBA was obtained by sequentially performing the steps shown in FIG. 2. The specific details will be described below.

First, the first acid precipitation step, the re-dissolution step, the second acid precipitation step, and the first cooling crystallization step were performed in the same manner as in Example 1.

Next, pure water (second aqueous solvent) was added to the first post-crystallization crystals recovered in the first cooling crystallization step to prepare a mixture at 13% by mass on a dry basis, and the obtained mixture was heated to 90°C. Next, the heated mixture was cooled to 25°C. As a result, second post-crystallization crystals were precipitated in the mixture. A cooling rate of the mixture was 0.4 °C/min. Thereafter, second post-crystallization crystals and a second waste liquid were recovered by solid-liquid separation (second cooling crystallization step).

Next, the recovered second post-crystallization crystals were heated and dried to obtain a powdery purified product (drying step).

9.5. Example 5

Among the steps shown in FIG. 5, each step using the starting materials in the second lot was sequentially performed to obtain a purified product consisting of purified 4HBA. The specific details will be described below.

First, the same step as the first acid precipitation step of Example 4 was performed to recover first post-acid-precipitation crystals.

Next, the same step as the re-dissolution step of Example 4 was performed to recover a solution. The first waste liquid recovered in the first cooling crystallization step of Example 4 was reused as the basic solvent. Specifically, by performing the reuse operation R1 shown in FIG. 5, the entire amount of the basic solvent, which was originally planned to be used in Example 5, excluding the base, was replaced with the first waste liquid.

Next, the same step as the second acid precipitation step of Example 4 was performed to recover second post-acid-precipitation crystals.

Next, the same step as the first cooling crystallization step of Example 4 was performed to recover first post-crystallization crystals.

Next, the same step as the second cooling crystallization step of Example 4 was performed to recover second post-crystallization crystals.

Next, the same step as the drying step of Example 4 was performed to obtain a powdery purified product.

9.6. Example 6

Among the steps shown in FIG. 5, each step using the starting materials in the second lot was sequentially performed to obtain a purified product consisting of purified 4HBA. The specific details will be described below.

First, the same step as the first acid precipitation step of Example 4 was performed to recover first post-acid-precipitation crystals.

Next, the same step as the re-dissolution step of Example 4 was performed to recover a solution.

Next, the same step as the second acid precipitation step of Example 4 was performed to recover second post-acid-precipitation crystals.

Next, the same step as the first cooling crystallization step of Example 4 was performed to recover first post-crystallization crystals. The second waste liquid recovered in the second cooling crystallization step of Example 4 was reused as the first aqueous solvent. Specifically, by performing the reuse operation R3 shown in FIG. 5, the entire amount of the first aqueous solvent, which was originally planned to be used in Example 6, was replaced with the second waste liquid.

Next, the same step as the second cooling crystallization step of Example 4 was performed to recover second post-crystallization crystals.

Next, the same step as the drying step of Example 4 was performed to obtain a powdery purified product.

9.7. Example 7

Among the steps shown in FIG. 5, each step using the starting materials in the second lot was sequentially performed to obtain a purified product consisting of purified 4HBA. The specific details will be described below.

First, the same step as the first acid precipitation step of Example 4 was performed to recover first post-acid-precipitation crystals.

Next, the same step as the re-dissolution step of Example 4 was performed to recover a solution. The first waste liquid recovered in the first cooling crystallization step of Example 4 was reused as the basic solvent. Specifically, by performing the reuse operation R1 shown in FIG. 5, the entire amount of the basic solvent, which was originally planned to be used in Example 7, excluding the base, was replaced with the first waste liquid.

Next, the same step as the second acid precipitation step of Example 4 was performed to recover second post-acid-precipitation crystals.

Next, the same step as the first cooling crystallization step of Example 4 was performed to recover first post-crystallization crystals. The second waste liquid recovered in the second cooling crystallization step of Example 4 was reused as the first aqueous solvent. Specifically, by performing the reuse operation R3 shown in FIG. 5, the entire amount of the first aqueous solvent, which was originally planned to be used in Example 7, was replaced with the second waste liquid.

Next, the same step as the second cooling crystallization step of Example 4 was performed to recover second post-crystallization crystals.

Next, the same step as the drying step of Example 4 was performed to obtain a powdery purified product.

### 9.8. Example 8

A purified product consisting of purified 4HBA was obtained by sequentially performing the steps shown in FIG. 3. The specific details will be described below.

First, the first acid precipitation step, the re-dissolution step, the second acid precipitation step, the first cooling crystallization step, and the second cooling crystallization step were performed in the same manner as in Example 4.

Next, pure water (third aqueous solvent) was added to the second post-crystallization crystals recovered in the second cooling crystallization step to prepare a mixture at 13% by mass on a dry basis, and the obtained mixture was heated to 90°C. Next, the heated mixture was cooled to 25°C. As a result, third post-crystallization crystals were precipitated in the mixture. A cooling rate of the mixture was 0.4 °C/min. Thereafter, third post-crystallization crystals and a third waste liquid were recovered by solid-liquid separation (third cooling crystallization step).

Next, the recovered third post-crystallization crystals were heated and dried to obtain a powdery purified product (drying step).

### 9.9. Example 9

Among the steps shown in FIG. 6, each step using the starting materials in the second lot was sequentially performed to obtain a purified product consisting of purified 4HBA. The specific details will be described below.

First, the same step as the first acid precipitation step of Example 8 was performed to recover first post-acid-precipitation crystals.

Next, the same step as the re-dissolution step of Example 8 was performed to recover a solution. The first waste liquid recovered in the first cooling crystallization step of Example 8 was reused as the basic solvent. Specifically, by performing the reuse operation R1 shown in FIG. 6, the entire amount of the basic solvent, which was originally planned to be used in Example 9, excluding the base, was replaced with the first waste liquid.

Next, the same step as the second acid precipitation step of Example 8 was performed to recover second post-acid-precipitation crystals.

Next, the same step as the first cooling crystallization step of Example 8 was performed to recover first post-crystallization crystals.

Next, the same step as the second cooling crystallization step of Example 8 was performed to recover second post-crystallization crystals.

Next, the same step as the third cooling crystallization step of Example 8 was performed to recover third post-crystallization crystals.

Next, the same step as the drying step of Example 8 was performed to obtain a powdery purified product.

### 9.10. Example 10

Among the steps shown in FIG. 6, each step using the starting materials in the second lot was sequentially performed to obtain a purified product consisting of purified 4HBA. The specific details will be described below.

First, the same step as the first acid precipitation step of Example 8 was performed to recover first post-acid-precipitation crystals.

Next, the same step as the re-dissolution step of Example 8 was performed to recover a solution.

Next, the same step as the second acid precipitation step of Example 8 was performed to recover second post-acid-precipitation crystals.

Next, the same step as the first cooling crystallization step of Example 8 was performed to recover first post-crystallization crystals. The second waste liquid recovered in the second cooling crystallization step of Example 8 was reused as the first aqueous solvent. Specifically, by performing the reuse operation R3 shown in FIG. 6, the entire amount of the first aqueous solvent, which was originally planned to be used in Example 10, was replaced with the second waste liquid.

Next, the same step as the second cooling crystallization step of Example 8 was performed to recover second post-crystallization crystals.

Next, the same step as the third cooling crystallization step of Example 8 was performed to recover third post-crystallization crystals.

Next, the same step as the drying step of Example 8 was performed to obtain a powdery purified product.

9.11. Example 11

Among the steps shown in FIG. 6, each step using the starting materials in the second lot was sequentially performed to obtain a purified product consisting of purified 4HBA. The specific details will be described below.

First, the same step as the first acid precipitation step of Example 8 was performed to recover first post-acid-precipitation crystals.

Next, the same step as the re-dissolution step of Example 8 was performed to recover a solution. The first waste liquid recovered in the first cooling crystallization step of Example 8 was reused as the basic solvent. Specifically, by performing the reuse operation R1 shown in FIG. 6, the entire amount of the basic solvent, which was originally planned to be used in Example 11, excluding the base, was replaced with the first waste liquid.

Next, the same step as the second acid precipitation step of Example 8 was performed to recover second post-acid-precipitation crystals.

Next, the same step as the first cooling crystallization step of Example 8 was performed to recover first post-crystallization crystals. The second waste liquid recovered in the second cooling crystallization step of Example 8 was reused as the first aqueous solvent. Specifically, by performing the reuse operation R3 shown in FIG. 6, the entire amount of the first aqueous solvent, which was originally planned to be used in Example 11, was replaced with the second waste liquid.

Next, the same step as the second cooling crystallization step of Example 8 was performed to recover second post-crystallization crystals.

Next, the same step as the third cooling crystallization step of Example 8 was performed to recover third post-crystallization crystals.

Next, the same step as the drying step of Example 8 was performed to obtain a powdery purified product.

9.12. Example 12

Among the steps shown in FIG. 6, each step using the starting materials in the second lot was sequentially performed to obtain a purified product consisting of purified 4HBA. The specific details will be described below.

First, the same step as the first acid precipitation step of Example 8 was performed to recover first post-acid-precipitation crystals.

Next, the same step as the re-dissolution step of Example 8 was performed to recover a solution. The first waste liquid recovered in the first cooling crystallization step of Example 8 was reused as the basic solvent. Specifically, by performing the reuse operation R1 shown in FIG. 6, the entire amount of the basic solvent, which was originally planned to be used in Example 12, excluding the base, was replaced with the first waste liquid.

Next, the same step as the second acid precipitation step of Example 8 was performed to recover second post-acid-precipitation crystals.

Next, the same step as the first cooling crystallization step of Example 8 was performed to recover first post-crystallization crystals. The second waste liquid recovered in the second cooling crystallization step of Example 8 was reused as the first aqueous solvent. Specifically, by performing the reuse operation R3 shown in FIG. 6, the entire amount of the first aqueous solvent, which was originally planned to be used in Example 12, was replaced with the second waste liquid.

Next, the same step as the second cooling crystallization step of Example 8 was performed to recover second post-crystallization crystals. The third waste liquid recovered in the third cooling crystallization step of Example 8 was reused as the second aqueous solvent. Specifically, by performing the reuse operation R4 shown in FIG. 6, the entire amount of the second aqueous solvent, which was originally planned to be used in Example 12, was replaced with the third waste liquid.

Next, the same step as the third cooling crystallization step of Example 8 was performed to recover third post-crystallization crystals.

Next, the same step as the drying step of Example 8 was performed to obtain a powdery purified product.

9.13. Comparative Example 1

Among the steps shown in FIG. 1, the same steps as the first acid precipitation step and the drying step were performed to recover a purified product.

9.14. Comparative Example 2

Among the steps shown in FIG. 1, the same steps as the first acid precipitation step, the first cooling crystallization step, and the drying step were performed to recover a purified product.

9.15. Comparative Example 3

Among the steps shown in FIG. 2, the same steps as the first acid precipitation step, the first cooling crystallization step, the second cooling crystallization step, and the drying step were performed to recover a purified product.

9.16. Comparative Example 4

Among the steps shown in FIG. 3, the same steps as the first acid precipitation step, the first cooling crystallization step, the second cooling crystallization step, the third cooling crystallization step, and the drying step were performed to recover a purified product.

9.17. Comparative Example 5

Among the steps shown in FIG. 3, the same steps as the first acid precipitation step, the re-dissolution step, the second acid precipitation step, and the drying step were performed to recover a purified product.

In Tables 1 and 2 described below, the presence or absence of each production step is indicated by "+" or "-". "+" indicates that the step was present, and "-" indicates that the step was not present.

In addition, in Tables 1 and 2, the presence or absence of the reuse of the waste liquid is indicated by "+" or "-". "+" indicates that the waste liquid was reused, and "-" indicates that the waste liquid was not reused.

10. Evaluation of purified product

10.1. Yield of 4HBA in purified product

The yield of 4HBA in the purified product was calculated based on the mass of 4HBA in the purified product obtained in each of Examples and Comparative Examples, and the mass of 4HBA in the starting material liquid. The following calculation expression was used for the calculation of the yield. The calculation results are shown in Tables 1 and 2.

Yield = (Mass of 4HBA in purified product)/(Mass of 4HBA in starting material liquid) $\times$ 100

10.2. Purity of 4HBA in purified product

The purity of 4HBA in the purified product obtained in each of Examples and Comparative Examples was calculated. The calculation results are shown in Tables 1 and 2.

10.3. Concentration of ionic impurities in purified product

The concentration of ionic impurities (sodium ions and sulfate ions) in the purified product obtained in each of Examples and Comparative Examples was measured. The measurement results are shown in Tables 1 and 2.

10.4. Coloring of purified product (absorbance of solution)

An absorbance of an ethanol solution of the purified product obtained in each of Examples and Comparative Examples was measured by the above-described method. The measurement results are shown in Tables 1 and 2.

10.5. Aerated bulk density of purified product

An aerated bulk density of the purified product obtained in each of Examples and Comparative Examples was measured. The measured aerated bulk density was evaluated according to the following evaluation standard. The evaluation results are shown in Tables 1 and 2.

A: high aerated bulk density (0.50 g/cm$^3$ or more)
B: slightly high aerated bulk density (0.45 g/cm$^3$ or more and less than 0.50 g/cm$^3$)
C: slightly low aerated bulk density (0.40 g/cm$^3$ or more and less than 0.45 g/cm$^3$)
D: low aerated bulk density (less than 0.40 g/cm$^3$)

10.6. Average crystallite diameter of purified product

An average crystallite diameter of the purified product obtained in each of Examples and Comparative Examples was measured. The measured average crystallite diameter was evaluated according to the following evaluation standard. The measurement results and the evaluation results are shown in Tables 1 and 2.

A: large average crystallite diameter (more than 40 nm)

B: slightly large average crystallite diameter (more than 36 nm and 40 nm or less)

C: slightly small average crystallite diameter (more than 32 nm and 36 nm or less)

D: small average crystallite diameter (32 nm or less)

[Table 1]

| | | | Example 1 | Example 2 | Example 3 | Example 4 | Example 5 | Example 6 | Example 7 | Example 8 | Example 9 |
|---|---|---|---|---|---|---|---|---|---|---|---|
| Production step | First acid precipitation step | | - | + | + | + | + | + | + | + | + | + |
| | Re-dissolution step | | - | + | + | + | + | + | + | + | + | + |
| | Second acid precipitation step | | - | + | + | + | + | + | + | + | + | + |
| | First cooling crystallization step | | - | + | + | + | + | + | + | + | + | + |
| | Second cooling crystallization step | | - | - | - | - | + | + | + | + | + | + |
| | Third cooling crystallization step | | - | - | - | - | - | - | - | - | + | + |
| | Drying step | | - | + | + | + | + | + | + | + | + | + |
| Reuse of waste liquid | Reuse of waste liquid in re-dissolution step | Presence or absence of euse | - | - | + | + | - | + | - | + | - | + |
| | | Kind of waste liquid | - | - | First waste liquid | First waste liquid | - | First waste liquid | - | First waste liquid | - | First waste liquid |
| | Reuse of waste liquid in first cooling crystallization step | Presence or bsence of reuse | - | - | - | + | - | - | + | + | - | - |
| | | Kind of waste liquid | - | - | - | First waste liquid | - | - | Second waste liquid | Second waste liquid | - | - |
| | Reuse of waste liquid in second cooling crystallization step | Presence or absence of reuse | - | - | - | - | - | - | - | - | - | - |
| | | Kind of waste liquid | - | - | - | - | - | - | - | - | - | - |

(continued)

| | | | Example 1 | Example 2 | Example 3 | Example 4 | Example 5 | Example 6 | Example 7 | Example 8 | Example 9 |
|---|---|---|---|---|---|---|---|---|---|---|---|
| Evaluation result of purified product | Yield of 4HBA | % | 80 | 81 | 83 | 74 | 75 | 81 | 82 | 71 | 72 |
| | Purity of 4HBA | % | 99 | 99 | 98 | Exceeding 99 | Exceeding 99 | Exceeding 99 | Exceeding 99 | Exceeding 99 | Exceeding 99 |
| | Concentration of sodium ion | ppm | 1,200 | 1,200 | 3,200 | 21 | 25 | 28 | 33 | 0.0 | 0.0 |
| | Concentration of sulfate ion | ppm | 3,300 | 3,400 | 6,300 | 57 | 70 | 60 | 63 | 0.0 | 0.0 |
| | Absorbance of ethanol solution | - | 0.018 | 0.020 | 0.020 | 0.008 | 0.010 | 0.008 | 0.009 | 0.004 | 0.006 |
| | Aerated bulk density | - | B | B | B | A | A | A | A | A | A |
| | Average crystallite diameter | Measured value | nm | 42 | 42 | 43 | 43 | 43 | 43 | 43 | 44 | 44 |
| | | Evaluation | - | A | A | A | A | A | A | A | A | A |

[Table 2]

| | | | | Example 10 | Example 11 | Example 12 | Comparative Example 1 | Comparative Example 2 | Comparative Example 3 | Comparative Example 4 | Comparative Example 5 |
|---|---|---|---|---|---|---|---|---|---|---|---|
| Production step | First acid precipitation step | | - | + | + | + | + | + | + | + | + |
| | Re-dissolution step | | - | + | + | + | - | - | - | - | + |
| | Second acid precipitation step | | - | + | + | + | - | - | - | - | + |
| | First cooling crystallization step | | - | + | + | + | - | + | + | + | - |
| | Second cooling crystallization step | | - | + | + | + | - | - | + | + | - |
| | Third cooling crystallization step | | - | + | + | + | - | - | - | + | - |
| | Drying step | | - | + | + | + | + | + | + | + | + |
| Reuse of waste liquid | Reuse of waste liquid in re- dissolution step | Presence or absence of reuse | - | - | + | + | - | - | - | - | - |
| | | Kind of waste liquid | | - | First waste liquid | First waste liquid | - | - | - | - |
| | Reuse of waste liquid in first cooling crystallization step | Presence or absence of reuse | - | + | + | + | - | - | - | - | - |
| | | Kind of waste liquid | | Second waste liquid | Second waste liquid | Second waste liquid | - | - | - | - | - |
| | Reuse of waste liquid in second cool-ing crystalliza-tion step | Presence on of reuse | - | - | - | + | - | - | - | - | - |
| | | Kind of waste liquid | | - | - | Third waste liquid | - | - | - | - | - |

(continued)

| | | Example 10 | Example 11 | Example 12 | Comparative Example 1 | Comparative Example 2 | Comparative Example 3 | Comparative Example 4 | Comparative Example 5 |
|---|---|---|---|---|---|---|---|---|---|
| Yield of 4HBA | % | 76 | 78 | 79 | 89 | 86 | 80 | 77 | 79 |
| Purity of 4HBA | % | Exceeding 99 | Exceeding 99 | Exceeding 99 | 92 | 96 | 98 | 99 | 92 |
| Evaluation result of purified product — Concentration of sodium ion | ppm | 0.6 | 0.0 | 1.4 | 8,500 | 2,700 | 133 | 20 | 24,000 |
| Concentration of sulfate ion | ppm | 0.0 | 6.0 | 3.0 | 17,000 | 5,300 | 150 | 27 | 11,000 |
| Absorbance of ethanol solution | - | 0.005 | 0.006 | 0.008 | 0.594 | 0.549 | 0.498 | 0.470 | 0.027 |
| Aerated bulk density | - | A | A | A | D | D | D | D | C |
| Average crystallite diameter — Measured value | nm | 44 | 44 | 44 | 38 | 32 | 36 | 42 | 40 |
| Average crystallite diameter — Evaluation | - | A | A | A | B | D | C | A | B |

As is clear from Tables 1 and 2, it was found that the purified product obtained in each of Examples was composed of 4HBA having high purity and less coloring. In addition, it was also found that, in each of Examples, the 4HBA could be recovered at a high yield of 70% or more. Furthermore, it was also found that, in a case where the waste liquid was reused, the yield and the purity could be further increased. In particular, the tendency was remarkable in a case where the step of reusing the waste liquid was divided for each type of the waste liquid.

From the above, it was clarified that, according to the present invention, 4HBA having high purity and less coloring could be produced at a high yield.

In addition, it was found that the purified product obtained in each of Examples had a higher aerated bulk density and a higher average crystallite diameter than those in each of Comparative Examples. In addition, as a reference example, in a case where an aerated bulk density of a reagent of 4HBA derived from petroleum was measured, the aerated bulk density was 0.44 g/cm$^3$.

INDUSTRIAL APPLICABILITY

According to the present invention, 4-hydroxybenzoic acid having high purity and less coloring can be produced at a high yield. Accordingly, the present invention has industrial applicability.

REFERENCE SIGNS LIST

22 Starting material liquid
24 Acid
26 First post-acid-precipitation crystal
28 Waste liquid after acid precipitation
41 Base
42 Basic solvent
44 Adsorbent
46 Waste adsorbent
48 Solution
64 Acid
66 Second post-acid-precipitation crystal
68 Waste liquid after acid precipitation
82 First aqueous solvent
86 First post-crystallization crystal
88 First waste liquid
102 Second aqueous solvent
106 Second post-crystallization crystal
108 Second waste liquid
122 Third aqueous solvent
126 Third post-crystallization crystal
128 Third waste liquid
142 Purified product
L1 First lot
L2 Second lot
P1 First unit process
P1-1 First unit process
P1-2 First unit process
P2 Second unit process
P2-1 Second unit process
P2-2 Second unit process
P3 Third unit process
P3-1 Third unit process
P3-2 Third unit process
R1 Reuse operation
R2 Reuse operation
R3 Reuse operation
R4 Reuse operation

S102 First acid precipitation step
S104 Re-dissolution step
S106 Second acid precipitation step
S108 First cooling crystallization step
S110 Second cooling crystallization step
S112 Third cooling crystallization step
S114 Drying step

**Claims**

1. A method for producing 4-hydroxybenzoic acid, comprising:

   a first acid precipitation step of mixing a starting material liquid containing a metal salt of 4-hydroxybenzoic acid and water with an acid to precipitate a first post-acid-precipitation crystal of 4-hydroxybenzoic acid;
   a re-dissolution step of mixing the first post-acid-precipitation crystal with a basic solvent to obtain a solution in which the first post-acid-precipitation crystal is re-dissolved, and adding an adsorbent to the solution;
   a second acid precipitation step of mixing the solution after the re-dissolution step with an acid to precipitate a second post-acid-precipitation crystal of 4-hydroxybenzoic acid; and
   a first cooling crystallization step of mixing the second post-acid-precipitation crystal with a first aqueous solvent and subsequently performing cooling crystallization to precipitate a first post-crystallization crystal of 4-hydroxybenzoic acid, and separating the first post-crystallization crystal and a first waste liquid.

2. The method for producing 4-hydroxybenzoic acid according to Claim 1,
   wherein, in a case where a process from the first acid precipitation step to the first cooling crystallization step is defined as a first unit process,

   the starting material liquid in a first lot is supplied to the first unit process, and subsequently
   the starting material liquid in a second lot is supplied to the first unit process,
   the first waste liquid obtained from the first unit process to which the starting material liquid in the first lot is supplied is reused as at least one of the basic solvent and the first aqueous solvent used in the first unit process to which the starting material liquid in the second lot is supplied.

3. The method for producing 4-hydroxybenzoic acid according to Claim 1, further comprising:
   a second cooling crystallization step of mixing the first post-crystallization crystal with a second aqueous solvent and subsequently performing cooling crystallization to precipitate a second post-crystallization crystal of 4-hydroxybenzoic acid, and separating the second post-crystallization crystal and a second waste liquid.

4. The method for producing 4-hydroxybenzoic acid according to Claim 3,
   wherein, in a case where a process from the first acid precipitation step to the second cooling crystallization step is defined as a second unit process,

   the starting material liquid in a first lot is supplied to the second unit process, and subsequently
   the starting material liquid in a second lot is supplied to the second unit process,
   the second waste liquid obtained from the second unit process to which the starting material liquid in the first lot is supplied is reused as at least one of the basic solvent and the first aqueous solvent used in the second unit process to which the starting material liquid in the second lot is supplied.

5. The method for producing 4-hydroxybenzoic acid according to Claim 4,

   wherein the first waste liquid obtained from the second unit process to which the starting material liquid in the first lot is supplied is reused as the basic solvent used in the second unit process to which the starting material liquid in the second lot is supplied, and
   the second waste liquid obtained from the second unit process to which the starting material liquid in the first lot is supplied is reused as the first aqueous solvent used in the second unit process to which the starting material liquid in the second lot is supplied.

6. The method for producing 4-hydroxybenzoic acid according to any one of Claims 3 to 5, further comprising:

a third cooling crystallization step of mixing the second post-crystallization crystal with a third aqueous solvent and subsequently performing cooling crystallization to precipitate a third post-crystallization crystal of 4-hydroxybenzoic acid, and separating the third post-crystallization crystal and a third waste liquid.

7. The method for producing 4-hydroxybenzoic acid according to Claim 6,
wherein, in a case where a process from the first acid precipitation step to the third cooling crystallization step is defined as a third unit process,

the starting material liquid in a first lot is supplied to the third unit process, and subsequently
the starting material liquid in a second lot is supplied to the third unit process,
the third waste liquid obtained from the third unit process to which the starting material liquid in the first lot is supplied is reused as at least one of the basic solvent, the first aqueous solvent, and the second aqueous solvent used in the third unit process to which the starting material liquid in the second lot is supplied.

8. The method for producing 4-hydroxybenzoic acid according to Claim 7,

wherein the first waste liquid obtained from the third unit process to which the starting material liquid in the first lot is supplied is reused as the basic solvent used in the third unit process to which the starting material liquid in the second lot is supplied,
the second waste liquid obtained from the third unit process to which the starting material liquid in the first lot is supplied is reused as the first aqueous solvent used in the third unit process to which the starting material liquid in the second lot is supplied, and
the third waste liquid obtained from the third unit process to which the starting material liquid in the first lot is supplied is reused as the second aqueous solvent used in the third unit process to which the starting material liquid in the second lot is supplied.

9. The method for producing 4-hydroxybenzoic acid according to any one of Claims 1 to 5,
wherein, in a case where the first post-crystallization crystal is mixed with ethanol to prepare an ethanol solution having a concentration of 20 g/L, an absorbance of the ethanol solution at a wavelength of 425 nm is 0.025 or less.

10. The method for producing 4-hydroxybenzoic acid according to any one of Claims 1 to 5,
wherein the adsorbent is activated carbon.

# FIG. 1

## FIG. 2

```
                                    ┌─────────┐
                                    │  START  │
                                    └────┬────┐
                                         │
┌───────────┐                       S102 │
│ STARTING  │                    ┌────────▼────────┐          ┌─────────────┐
22─│ MATERIAL  │─────────────────►│   FIRST ACID    │─────────►│ WASTE LIQUID│─28
│  LIQUID   │                    │PRECIPITATION STEP│          │ AFTER ACID  │
└───────────┘                    └─────────────────┘          │PRECIPITATION│
                                                               └─────────────┘
┌───────────┐                                    ┌──────────────────┐
24─│   ACID    │                                 │  FIRST POST-ACID- │─26
└───────────┘                              S104  │PRECIPITATION CRYSTAL│
┌───────────┐                    ┌─────────▼────────┐          └──────────────────┘
42─│  BASIC    │─────────────────►│                  │          ┌─────────┐
│  SOLVENT  │                    │ RE-DISSOLUTION   │─────────►│  WASTE  │─46
└───────────┘                    │      STEP        │          │ADSORBENT│
┌───────────┐                    └──────────────────┘          └─────────┘
44─│ ADSORBENT │
└───────────┘                              ┌──────────┐
                                           │ SOLUTION │─48
                                           └──────────┘
                                     S106
                                    ┌─────────▼────────┐          ┌─────────────┐
┌───────────┐                       │  SECOND ACID     │─────────►│ WASTE LIQUID│─68
64─│   ACID    │─────────────────────►│PRECIPITATION STEP│          │ AFTER ACID  │
└───────────┘                       └──────────────────┘          │PRECIPITATION│
                                                                  └─────────────┘
                                           ┌──────────────────┐
                                     S108  │  SECOND POST-ACID-│─66
┌───────────┐                              │PRECIPITATION CRYSTAL│
│  FIRST    │                    ┌─────────▼────────┐          └──────────────────┘
82─│ AQUEOUS   │─────────────────►│  FIRST COOLING   │─────────►┌─────────┐
│  SOLVENT  │                    │CRYSTALLIZATION STEP│          │  FIRST  │─88
└───────────┘                    └──────────────────┘          │WASTE LIQUID│
                                                               └─────────┘
                                           ┌──────────────────┐
                                     S110  │   FIRST POST-     │─86
┌───────────┐                              │CRYSTALLIZATION CRYSTAL│
│  SECOND   │                    ┌─────────▼────────┐          └──────────────────┘
102─│ AQUEOUS   │─────────────────►│  SECOND COOLING  │─────────►┌─────────┐
│  SOLVENT  │                    │CRYSTALLIZATION STEP│          │ SECOND  │─108
└───────────┘                    └──────────────────┘          │WASTE LIQUID│
                                                               └─────────┘
                                           ┌──────────────────┐
                                     S114  │  SECOND POST-     │─106
                                    ┌─────────▼────────┐       │CRYSTALLIZATION CRYSTAL│
                                    │                  │       └──────────────────┘
                                    │   DRYING STEP    │
                                    └──────────────────┘
                                           ┌──────────┐
                                           │ PURIFIED │─142
                                           │ PRODUCT  │
                                           └──────────┘
                                         ┌────▼────┐
                                         │   END   │
                                         └─────────┘
```

## FIG. 3

START

**22** — STARTING MATERIAL LIQUID

**24** — ACID

S102 — FIRST ACID PRECIPITATION STEP

**28** — WASTE LIQUID AFTER ACID PRECIPITATION

**26** — FIRST POST-ACID-PRECIPITATION CRYSTAL

**42** — BASIC SOLVENT

**44** — ADSORBENT

S104 — RE-DISSOLUTION STEP

**46** — WASTE ADSORBENT

**48** — SOLUTION

**64** — ACID

S106 — SECOND ACID PRECIPITATION STEP

**68** — WASTE LIQUID AFTER ACID PRECIPITATION

**66** — SECOND POST-ACID-PRECIPITATION CRYSTAL

**82** — FIRST AQUEOUS SOLVENT

S108 — FIRST COOLING CRYSTALLIZATION STEP

**88** — FIRST WASTE LIQUID

**86** — FIRST POST-CRYSTALLIZATION CRYSTAL

**102** — SECOND AQUEOUS SOLVENT

S110 — SECOND COOLING CRYSTALLIZATION STEP

**108** — SECOND WASTE LIQUID

**106** — SECOND POST-CRYSTALLIZATION CRYSTAL

**122** — THIRD AQUEOUS SOLVENT

S112 — THIRD COOLING CRYSTALLIZATION STEP

**128** — THIRD WASTE LIQUID

**126** — THIRD POST-CRYSTALLIZATION CRYSTAL

S114 — DRYING STEP

**142** — PURIFIED PRODUCT

END

FIG. 4

**FIRST LOT L1**

START  — P1-1(P1)

22 — STARTING MATERIAL LIQUID

24 — ACID

S102 — FIRST ACID PRECIPITATION STEP → WASTE LIQUID AFTER ACID PRECIPITATION ~28

FIRST POST-ACID-PRECIPITATION CRYSTAL ~26

42 — BASIC SOLVENT

44 — ADSORBENT

S104 — RE-DISSOLUTION STEP → WASTE ADSORBENT ~46

S106 — SOLUTION ~48

64 — ACID

SECOND ACID PRECIPITATION STEP → WASTE LIQUID AFTER ACID PRECIPITATION ~68

SECOND POST-ACID-PRECIPITATION CRYSTAL ~66

82 — FIRST AQUEOUS SOLVENT

S108 — FIRST COOLING CRYSTALLIZATION STEP → FIRST WASTE LIQUID ~88

FIRST POST-CRYSTALLIZATION CRYSTAL ~86

S114 — DRYING STEP

PURIFIED PRODUCT ~142

END

R1

R2

**SECOND LOT L2**

START — P1-2(P1)

22 — STARTING MATERIAL LIQUID

24 — ACID

S102 — FIRST ACID PRECIPITATION STEP → WASTE LIQUID AFTER ACID PRECIPITATION ~28

FIRST POST-ACID-PRECIPITATION CRYSTAL ~26

41 — BASE

44 — ADSORBENT

S104 — RE-DISSOLUTION STEP → WASTE ADSORBENT ~46

S106 — SOLUTION ~48

64 — ACID

SECOND ACID PRECIPITATION STEP → WASTE LIQUID AFTER ACID PRECIPITATION ~68

SECOND POST-ACID-PRECIPITATION CRYSTAL ~66

S108 — FIRST COOLING CRYSTALLIZATION STEP → FIRST WASTE LIQUID ~88

FIRST POST-CRYSTALLIZATION CRYSTAL ~86

S114 — DRYING STEP

PURIFIED PRODUCT ~142

END

FIG. 5

EP 4 703 344 A1

FIG. 6

EP 4 703 344 A1

## INTERNATIONAL SEARCH REPORT

| International application No. |
| --- |
| **PCT/JP2024/014252** |

---

**A.    CLASSIFICATION OF SUBJECT MATTER**

*C07C 51/487*(2006.01)i; *C07C 51/43*(2006.01)i; *C07C 65/03*(2006.01)i
FI:    C07C51/487; C07C51/43; C07C65/03 B

According to International Patent Classification (IPC) or to both national classification and IPC

---

**B.    FIELDS SEARCHED**

Minimum documentation searched (classification system followed by classification symbols)

C07B31/00-61/00; C07B63/00-63/04; C07C1/00-409/44; C12P1/00-41/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII), CAplus/REGISTRY (STN)

---

**C.    DOCUMENTS CONSIDERED TO BE RELEVANT**

| Category* | Citation of document, with indication, where appropriate, of the relevant passages | Relevant to claim No. |
| --- | --- | --- |
| A | JP 6-78780 A (SHOWA DENKO K.K.) 22 March 1994 (1994-03-22) <br> claims 1-2, paragraphs [0003], [0037]-[0039] | 1-10 |
| A | JP 2021-38156 A (SUMITOMO BAKELITE CO., LTD.) 11 March 2021 (2021-03-11) <br> claims 1-5, paragraphs [0007]-[0008], [0023], [0038]-[0048], examples | 1-10 |
| A | WO 2004/092107 A1 (KABUSHIKI KAISHA UENO SEIYAKU OYO KENKYUJO) 28 October 2004 (2004-10-28) <br> claims 1-10, page 2, lines 14-15, page 4, line 8 to page 5, line 26, examples | 1-10 |
| A | CN 115181020 A (SHANGHAI CHEMICAL TECH. CO., LTD.) 14 October 2022 (2022-10-14) <br> claims 1-2, 7-10, paragraphs [0003], [0022]-[0026], examples | 1-10 |
| A | JP 62-106046 A (RHONE POULENC SPEC. CHIM.) 16 May 1987 (1987-05-16) <br> claim 1, page 2, upper right column, line 17 to lower left column, line 7, page 5, upper right column, lines 6-13, examples | 1-10 |

---

☑ Further documents are listed in the continuation of Box C.        ☑ See patent family annex.

| | |
| --- | --- |
| \*       Special categories of cited documents: <br> "A"   document defining the general state of the art which is not considered to be of particular relevance <br> "D"   document cited by the applicant in the international application <br> "E"   earlier application or patent but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| Date of the actual completion of the international search | Date of mailing of the international search report |
| --- | --- |
| **12 June 2024** | **25 June 2024** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

**EP 4 703 344 A1**

## INTERNATIONAL SEARCH REPORT

International application No.

**PCT/JP2024/014252**

**C. DOCUMENTS CONSIDERED TO BE RELEVANT**

| Category* | Citation of document, with indication, where appropriate, of the relevant passages | Relevant to claim No. |
|---|---|---|
| A | JP 5-997 A (KAWASAKI STEEL CORP.) 08 January 1993 (1993-01-08)<br>claims 1, 5, paragraphs [0008]-[0010], [0033]-[0050], examples | 1-10 |
| A | JP 4-226938 A (KAWASAKI STEEL CORP.) 17 August 1992 (1992-08-17)<br>claims 1-6, paragraphs [0007]-[0013], [0031], [0042]-[0045], [0048]-[0050], examples | 1-10 |
| A | JP 59-27850 A (NIPPON KAYAKU KABUSHIKI KAISHA) 14 February 1984 (1984-02-14)<br>claim 1, example 8 | 1-10 |

Form PCT/ISA/210 (second sheet) (July 2022)

## INTERNATIONAL SEARCH REPORT
### Information on patent family members

| International application No. |
|---|
| **PCT/JP2024/014252** |

| Patent document cited in search report | | | Publication date (day/month/year) | Patent family member(s) | | | Publication date (day/month/year) |
|---|---|---|---|---|---|---|---|
| JP | 6-78780 | A | 22 March 1994 | (Family: none) | | | |
| JP | 2021-38156 | A | 11 March 2021 | (Family: none) | | | |
| WO | 2004/092107 | A1 | 28 October 2004 | US | 2006/0264670 | A1 | |
| | | | | claims 1-10, paragraphs [0008], [0025]-[0036], examples | | | |
| | | | | EP | 1614673 | A1 | |
| | | | | CN | 1805918 | A | |
| CN | 115181020 | A | 14 October 2022 | (Family: none) | | | |
| JP | 62-106046 | A | 16 May 1987 | US | 4814498 | A | |
| | | | | claim 1, column 1, lines 12-16, line 64 to column 2, line 3, column 5, lines 1-7, examples | | | |
| | | | | EP | 223723 | A1 | |
| | | | | KR | 10-1987-0003966 | A | |
| JP | 5-997 | A | 08 January 1993 | (Family: none) | | | |
| JP | 4-226938 | A | 17 August 1992 | (Family: none) | | | |
| JP | 59-27850 | A | 14 February 1984 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021038156 A